(19) Europäisches Patentamt
European Patent Office
Office européen des brevets

(11) EP 2 716 629 A1

(12) **EUROPEAN PATENT APPLICATION**
published in accordance with Art. 153(4) EPC

(43) Date of publication:
**09.04.2014 Bulletin 2014/15**

(21) Application number: **12793740.7**

(22) Date of filing: **29.03.2012**

(51) Int Cl.:
***C07D 207/16*** *(2006.01)* ***C12P 41/00*** *(2006.01)*

(86) International application number:
**PCT/JP2012/058452**

(87) International publication number:
**WO 2012/165023 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2011 JP 2011122587**

(71) Applicant: **API Corporation**
**Chuo-ku, Tokyo 103-0027 (JP)**

(72) Inventors:
- **UEHARA, Hisatoshi**
  **Yokohama-shi**
  **Kanagawa 227-8502 (JP)**
- **MIYAKE, Ryoma**
  **Yokohama-shi**
  **Kanagawa 227-8502 (JP)**
- **BANDO, Keisuke**
  **Yokohama-shi**
  **Kanagawa 227-8502 (JP)**
- **KAWABATA, Hiroshi**
  **Yokohama-shi**
  **Kanagawa 227-8502 (JP)**
- **MAEDA, Tomoko**
  **Yokohama-shi**
  **Kanagawa 227-8502 (JP)**

(74) Representative: **Godemeyer Blum Lenze - werkpatent**
**An den Gärten 7**
**51491 Overath (DE)**

# (54) METHOD FOR PRODUCING OPTICALLY ACTIVE ALPHA-SUBSTITUTED PROLINE

(57) The present invention aims to provide an industrial method practically suitable for producing optically active α-substituted prolines from an acyclic ketone compound by a small number of steps under mild conditions. The present invention relates to a production method of an optically active α-substituted proline (4) and/or an optically active α-substituted prolinamide (5), including (a) reacting an acyclic ketone compound (1) with at least one selected from ammonia, an ammonium salt, primary amine and a salt of primary amine, and a cyanating agent to give a cyclic nitrogen-containing compound (2), (b) hydrating the cyclic nitrogen-containing compound (2) to give an α-substituted prolinamide (3), and (c) resolving the α-substituted prolinamide (3) by one or more of (d) enzymatical hydrolysis, (e) resolution by diastereomeric salt formation, and (f) separation by column chromatography.

EP 2 716 629 A1

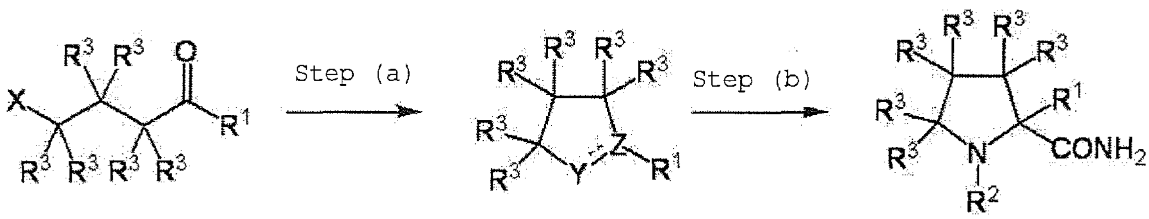


(1) (2) (3)

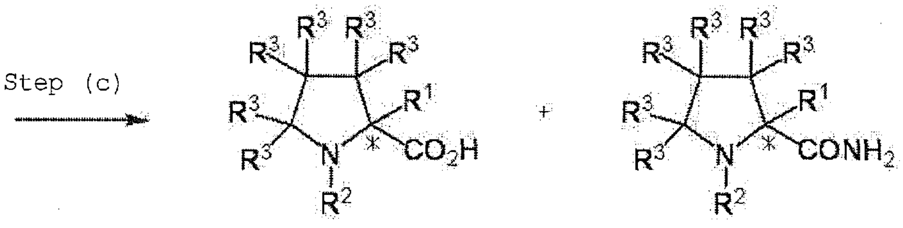


(4) (5)

## Description

### Technical Field

The present invention relates to an industrial method for producing optically active α-substituted proline from an acyclic ketone compound. An optically active α-substituted proline produced by the present invention is a compound useful for peptide structural chemistry, or as a pharmaceutical intermediate.

### Background Art

Optically active α-substituted prolines are considered to produce only a peptide having a low rotational degree of freedom and a limited conformation, since they tolerate only highly limited torsion angles in peptides containing them, and are drawing much attention in recent years (see, for example, non-patent document 1). Moreover, since they have structures with less fluctuation, they are considered to be useful as partial structures of highly selective pharmaceutical products, and have been actively utilized for drug discovery studies.

As a synthesis method of optically active α-substituted prolines, a method using L-proline as a starting material, which includes protecting amino acid with pivalaldehyde, and performing alkylation using a strong base and an alkylating agent is known (see, for example, non-patent document 2). An improved method thereof is a method using chloral instead of pivalaldehyde (see, for example, non-patent document 3). However, the both methods require use of an expensive strong base such as LDA and the like at an extremely low temperature of -78°C, which is not industrially suitable. These methods are also problematic in that only α-substituted proline in an S form can be produced from economical L-proline.

A method of synthesizing an optically active α-substituted proline by an intramolecular cyclization reaction using an amino acid such as L-alanine and the like as a starting material is also known (see, for example, non-patent document 4, patent document 1). However, the method is problematic in that an expensive strong base such as potassium hexamethyldisilazide, lithium hexamethyldisilazide and the like is required in the intramolecular cyclization reaction step. On the other hand, a method of performing a similar reaction by using a low cost potassium hydroxide has been reported (see non-patent document 5); however, it requires an industrially difficult operation of powderizing potassium hydroxide, and a problem of low producibility resulting from the use of 30-fold volume of DMSO as a solvent remains. In all cases, carboxyl group and amino group need to be protected in the intramolecular cyclization reaction step, which defectively increases the total production steps due to the protection and deprotection.

A catalytic asymmetric synthesis method using an optically active quaternary ammonium salt is also known (see, for example, non-patent document 6). Although the method shows very high stereoselectivity and yield, it requires use of tertiary butyl ester as a substrate, iodide as an alkylating agent, and cesium hydroxide as a base. These materials are all industrially expensive, and this method is not suitable for pharmaceutical or agrochemical intermediates desired to be produced at a low cost.

On the other hand, a method for obtaining optically active α-methylproline and optically active α-methylprolinamide by enzymatically resolving racemate of α-methylprolinamide is also known (see non-patent document 1). In this enzymatic resolution, Ochrobactrum anthropi NCIMB40321 and Mycobacterium neoaurum ATCC25975 show an E value to be a selectivity index of 317 and 240, respectively, and the enzymatic resolution itself is comparatively efficient, though synthesis of the racemate of α-methylprolinamide, which is a starting material of resolution, requires multisteps. To be specific, 4 steps of imine formation by alaninamide and benzaldehyde, addition to acrylonitrile by using sodium hydride, hydrolysis of imine, and hydrogenation are necessary, which is not preferable as a production method aiming at an industrial production at a low cost. Here, the E value is an index calculated by the following formula from the conversion ratio (c) of the reaction and optical purity (eeS) of the residual substrate.

$$E=\ln[(1-c)(1-eeS)]/\ln[(1-c)(1+eeS)]$$

A practical production method of the racemate of α-methylprolinamide other than this method is not known, and the development of an industrially superior production method of the racemate of α-substituted prolinamide has been desired.

As a method of producing the racemate of α-substituted prolinamide by a small number of steps, a synthetic method by hydration of 2-cyano-2-substituted pyrrolidine is considered. Among the 2-cyano-2-substituted pyrrolidines to be a key to the method, 2-cyano-2-methylpyrrolidine is a known compound (see patent document 2). However, the production method thereof has not been reported, and a practical production method is not known. As a similar compound, a synthetic example of N-(1-phenylethyl)-2-cyano-2-methylpyrrolidine has been reported (see non-patent document 7).

However, it requires specific aminonitrile, and the object product is obtained only in a low yield of 13%. On the other hand, in a different article by the same author (see non-patent document 8), 2-cyanopyrrolidine having a bicyclo skeleton is obtained in comparatively high yields (maximum 80%). The difference in the results shows that 2-cyanopyrrolidine is obtained in a high yield in a system where the bicyclo skeleton suppresses elimination of cyano group, but when such stabilization is absent, it is difficult to obtain 2-cyanopyrrolidine in a high yield. In addition, a synthetic example of 2-cyanopyrrolidine having a bicyclo skeleton from 1,7-dichloro-4-heptanone is also reported (see non-patent document 9). It is also evident in this example that the bicyclo skeleton is essential for achieving a high yield. This report states that the side reaction is remarkable in a reaction using potassium cyanide in a solvent containing water, and a non-aqueous reaction using acetone cyanohydrin or 2-amino-2-methylpropanenitrile is necessary. However, they have a risk of generating a hydrocyanic acid gas by thermal decomposition and the like, and the method is not an industrially preferable production method.

Pyrrolines, particularly 2-methylpyrroline, are highly useful compounds also sold as reagents. However, they are expensive and an economical method of industrial production has not been reported to date. For example, a production method of 2-methylpyrroline using 5-chloro-2-pentanone as a starting material is known (for example, non-patent document 10). However, since it requires 2-step reaction including substitution of chlorine atom by azide and the like, and cyclization while reducing with triphenylphosphine and the like, and produces a large amount of waste, it is not an industrially preferable method. On the other hand, a production method of 2-methylthiazolines using chloroacetone as a starting material is known (see, for example, patent document 3). However, 5-chloro-2-pentanone is known to be easily converted to cyclopropyl methyl ketone under basic conditions (see, for example, non-patent document 11). When the present inventors tried the method of patent document 3 using aqueous ammonia, a large amount of a cyclopropyl methyl ketone byproduct was observed. Therefore, the method of patent document 3 cannot be used as a production method of 2-methylpyrroline.

Document List

patent documents

patent document 1: WO2006/110816
patent document 2: JP-A-S49-31614
patent document 3: W02004/090152

non-patent documents

non-patent document 1: Chem. Eur. J., 2009, 15, 8015.
non-patent document 2: Org. Synth., 1995, 72, 62.
non-patent document 3: Synlett, 1999, 33.
non-patent document 4: J. Am. Chem. Soc., 2006, 128, 15394.
non-patent document 5: J. Am. Chem. Soc., 2008, 130, 4153.
non-patent document 6: Tetrahedron, 2010, 66, 4900.
non-patent document 7: Tetrahedron Asym., 2007, 18, 290.
non-patent document 8: Tetrahedron Asym., 2006, 17, 252.
non-patent document 9: Heterocycles, 1997, 45, 1447.
non-patent document 10: Bull. Soc. Chim. Fr., 1986, 83.
non-patent document 11: Org. Synth., 1951, 31, 74.

Summary of the Invention

Problems to be Solved by the Invention

The present invention aims to provide an industrial method practically suitable for producing optically active α-substituted prolines from an acyclic ketone compound by a small number of steps under mild conditions.

Means of Solving the Problems

The present inventors have conducted intensive studies in an attempt to solve the aforementioned problems and found that an optically active α-substituted proline and/or an optically active α-substituted prolinamide can be obtained by reacting an acyclic ketone compound with at least one selected from ammonia, an ammonium salt, primary amine and a salt of primary amine, and a cyanating agent to give a cyclic nitrogen-containing compound, hydrating the cyclic nitrogen-containing compound to give an α-substituted prolinamide, and resolving the α-substituted prolinamide, which resulted in the completion of the present invention.

According to the present invention, the following invention is provided.

[1] A method of producing an optically active α-substituted proline represented by the formula (4)

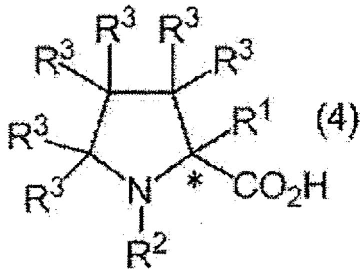
(4)

wherein $R^1$ is an optionally substituted alkyl group, an optionally substituted aryl group, or an optionally substituted heteroaryl group, $R^2$ is a hydrogen atom, an optionally substituted alkyl group, or an amino-protecting group, each $R^3$ is independently a hydrogen atom, an optionally substituted alkyl group, an optionally substituted aryl group, an optionally substituted heteroaryl group, an optionally substituted hydroxyl group, an optionally substituted amino group, an optionally substituted thiol group, or a halogen atom, two or more $R^3$ optionally form one or plural cyclic structures, and * shows an asymmetric carbon, or a salt thereof, and/or an optically active α-substituted prolinamide represented by the formula (5)

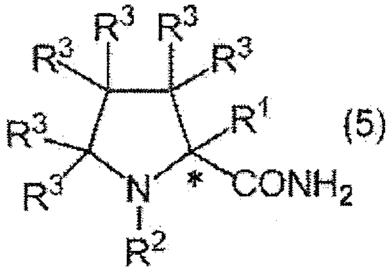
(5)

wherein each symbol is as defined above, or a salt thereof, comprising the following steps (a) to (c);

(a) reacting an acyclic ketone compound represented by the formula (1)

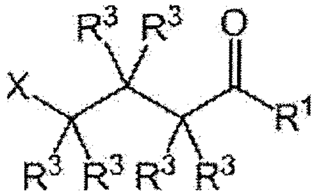
(1)

wherein $R^1$ and $R^3$ are as defined above, and X is a halogen atom or a sulfonyloxy group, with at least one selected from ammonia, an ammonium salt, primary amine and a salt of primary amine, and a cyanating agent and, where necessary, protecting a nitrogen atom on the pyrrolidine ring to give a cyclic nitrogen-containing compound represented by the formula (2)

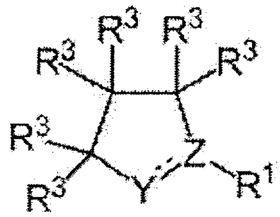

(2)

wherein $R^1$ and $R^3$ are as defined above, Y is a nitrogen atom or a nitrogen atom substituted by $R^2$, Z is a carbon atom or a carbon atom substituted by a cyano group, when Y is a nitrogen atom and Z is a carbon atom, then the bond between Y and Z is a double bond, when Y is a nitrogen atom substituted by $R^2$ and Z is a carbon atom substituted by a cyano group, then the bond between Y and Z is a single bond, and $R^2$ is as defined above, or a salt thereof; and

(b) hydrating the cyclic nitrogen-containing compound represented by the formula (2) or a salt thereof to give an $\alpha$-substituted prolinamide represented by the formula (3)

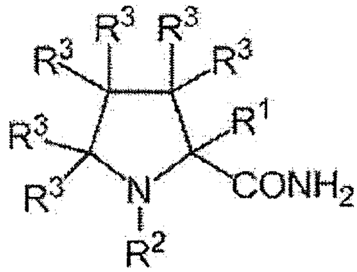

(3)

wherein each symbol is as defined above, or a salt thereof; and (c) resolving the $\alpha$-substituted prolinamide represented by the formula (3) or a salt thereof to give an optically active $\alpha$-substituted proline represented by the formula (4) or a salt thereof, and/or an optically active $\alpha$-substituted prolinamide represented by the formula (5) or a salt thereof.

[2] A method of producing a 2-substituted prolinamide represented by the formula (3)

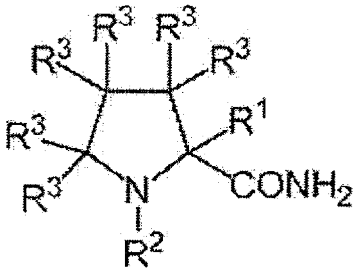

(3)

wherein each symbol is as defined in the aforementioned [1], or a salt thereof, comprising the following steps (a) and (b);

(a) reacting an acyclic ketone compound represented by the formula (1)

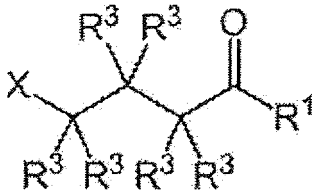

(1)

wherein each symbol is as defined in the aforementioned [1], with at least one selected from ammonia, an ammonium salt, primary amine and a salt of primary amine, and a cyanating agent and, where necessary,

protecting a nitrogen atom on the pyrrolidine ring to give a cyclic nitrogen-containing compound represented by the formula (2)

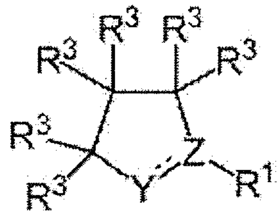

(2)

wherein each symbol is as defined in the aforementioned [1], or a salt thereof; and

(b) hydrating the cyclic nitrogen-containing compound represented by the formula (2) or a salt thereof to give an $\alpha$-substituted prolinamide represented by the formula (3) or a salt thereof.

[3] A method of producing a cyclic nitrogen-containing compound represented by the formula (2)

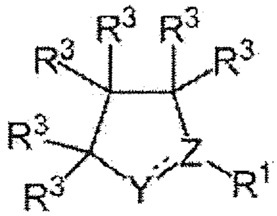

(2)

wherein each symbol is as defined in the aforementioned [1], or a salt thereof, comprising the following step (a); (a) reacting an acyclic ketone compound represented by the formula (1)

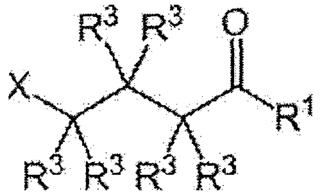

(1)

wherein each symbol is as defined in the aforementioned [1], with at least one selected from ammonia, an ammonium salt, primary amine and a salt of primary amine, and a cyanating agent and, where necessary, protecting a nitrogen atom on the pyrrolidine ring to give a cyclic nitrogen-containing compound represented by the formula (2) or a salt thereof.

[4] A method of producing an optically active $\alpha$-substituted proline represented by the formula (4)

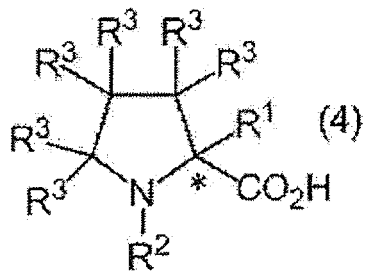

(4)

wherein each symbol is as defined in the aforementioned [1], or a salt thereof, and/or an optically active $\alpha$-substituted prolinamide represented by the formula (5)

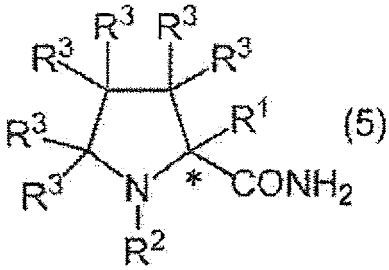

(5)

wherein each symbol is as defined in the aforementioned [1], or a salt thereof, comprising the following step (c);

(c) resolving an α-substituted prolinamide represented by the formula (3)

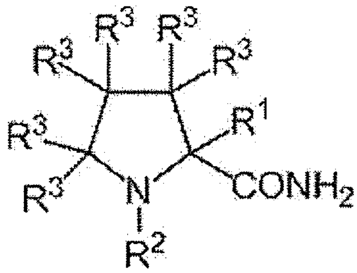

(3)

wherein each symbol is as defined in the aforementioned [1], or a salt thereof, to give an optically active α-substituted proline represented by the formula (4) or a salt thereof, and/or an optically active α-substituted prolinamide represented by the formula (5) or a salt thereof; wherein the resolution is any of the following steps (d) to (f):
(d) asymmetric hydrolysis of the amido group by an enzyme having an amidase activity derived from Rhizopus oryzae,
(e) resolution by diastereomeric salt formation,
(f) separation by column chromatography.

[5] An α-methylprolinamide represented by the formula (8)

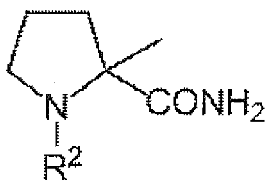

(8)

wherein $R^2$ is 1-phenylethyl group, 1-(1-naphthyl)ethyl group, 1-(2-naphthyl)ethyl group, or carbamoylphenylmethyl group, or a salt thereof.

Effect of the Invention

According to the present invention, an optically active α-substituted proline and/or an optically active α-substituted prolinamide useful for peptide structural chemistry or as key synthetic intermediates for pharmaceutical products can be produced efficiently from economical and easily available known compounds.

Description of Embodiments

In the present invention, $R^1$ is an optionally substituted alkyl group, an optionally substituted aryl group, or an optionally substituted heteroaryl group.

Examples of the "alkyl group" of the "optionally substituted alkyl group" include a straight chain, branched chain or cyclic alkyl group having 1 - 10 carbon atoms, for example, a straight chain alkyl group having 1 - 10 carbon atoms such as methyl group, ethyl group, n-propyl group, n-butyl group, n-pentyl group, n-hexyl group, n-heptyl group, n-octyl

group, n-nonyl group, n-decyl group and the like; a branched chain alkyl group having 3 - 10 carbon atoms such as isopropyl group, 1-methylpropyl group, t-butyl group and the like; and a cyclic alkyl group having 3 - 10 carbon atoms such as cyclopropyl group, cyclopentyl group, cyclohexyl group and the like.

Examples of the substituent that the alkyl group optionally has include a fluorine atom; an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from an alkyl group having 1 - 6 carbon atoms (e.g., methyl group, ethyl group, isopropyl group etc.), a halogen atom (e.g., fluorine atom, chlorine atom, bromine atom, iodine atom), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

As the substituted alkyl group, benzyl group, 4-methoxybenzyl group, allyl group, 2-fluoroethyl group and the like can be specifically mentioned.

Examples of the "aryl group" of the "optionally substituted aryl group" include an aromatic hydrocarbon group having 6 - 10 carbon atoms, for example, phenyl group, 1-naphthyl group, 2-naphthyl group and the like can be mentioned.

Examples of the substituent that the aryl group optionally has include a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from a halogen atom (e.g., fluorine atom, chlorine atom, bromine atom, iodine atom), an alkyl group having 1 - 6 carbon atoms (e.g., methyl group, ethyl group, isopropyl group etc.), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

Examples of the "heteroaryl group" of the "optionally substituted heteroaryl group" include a 5- or 6-membered aromatic heterocyclic group containing, as a ring-constituting atom besides carbon atoms, 1 to 4 heteroatoms selected from a nitrogen atom, an oxygen atom and a sulfur atom, for example, pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), pyrazolyl (e.g., 1-pyrazolyl, 3-pyrazolyl, 4-pyrazolyl), imidazolyl (e.g., 1-imidazolyl, 2-imidazolyl, 4-imidazolyl), isoxazolyl (e.g., 3-isoxazolyl, 4-isoxazolyl, 5-isoxazolyl), oxazolyl (e.g., 2-oxazolyl, 4-oxazolyl, 5-oxazolyl), isothiazolyl (e.g., 3-isothiazolyl, 4-isothiazolyl, 5-isothiazolyl), thiazolyl (e.g., 2-thiazolyl, 4-thiazolyl, 5-thiazolyl), triazolyl (1,2,3-triazol-4-yl, 1,2,4-triazol-3-yl), oxadiazolyl (1,2,4-oxadiazol-3-yl, 1,2,4-oxadiazol-5-yl), thiadiazolyl (1,2,4-thiadiazol-3-yl, 1,2,4-thiadiazol-5-yl), tetrazolyl, pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl, 3-pyrazinyl) and the like.

Examples of the substituent that the heteroaryl group optionally has include a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from a halogen atom (e.g., fluorine atom, chlorine atom, bromine atom, iodine atom), an alkyl group having 1 - 6 carbon atoms (e.g., methyl group, ethyl group, isopropyl group etc.), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

Of these, $R^1$ is preferably an optionally substituted alkyl group, more preferably methyl group, ethyl group, n-propyl group, n-butyl group, benzyl group or allyl group, particularly preferably methyl group showing the property of $\alpha$-substituted proline with the simplest structure.

In the present invention, $R^2$ is a hydrogen atom, an optionally substituted alkyl group, or an amino-protecting group.

Examples of the "alkyl group" of the "optionally substituted alkyl group" include a straight chain, branched chain or cyclic alkyl group having 1 - 10 carbon atoms, for example, a straight chain alkyl group having 1 - 10 carbon atoms such as methyl group, ethyl group, n-propyl group, n-butyl group, n-pentyl group, n-hexyl group, n-heptyl group, n-octyl group, n-nonyl group, n-decyl group and the like; a branched chain alkyl group having 3 - 10 carbon atoms such as isopropyl group, 1-methylpropyl group, t-butyl group and the like; and a cyclic alkyl group having 3 - 10 carbon atoms such as cyclopropyl group, cyclopentyl group, cyclohexyl group and the like.

Examples of the substituent that the alkyl group optionally has include a halogen atom (e.g., fluorine atom, chlorine atom, bromine atom, iodine atom); an alkenyl group having 2 - 6 carbon atoms (e.g., vinyl group etc.); an alkoxy

group having 1 - 6 carbon atoms (e.g., methoxy group, ethoxy group etc.); an alkoxycarbonyl group having 2 - 6 carbon atoms (e.g., methoxycarbonyl group, ethoxycarbonyl group etc.); carbamoyl group; carboxyl group; an aryl group having 6 - 10 carbon atoms (e.g., phenyl group, naphthyl group etc.) optionally having 1 to 3 substituents selected from an alkyl group having 1 - 6 carbon atoms (e.g., methyl group, ethyl group, isopropyl group etc.), a halogen atom (e.g., fluorine atom, chlorine atom, bromine atom, iodine atom), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different. When the optionally substituted alkyl group has an asymmetric center, it may be an R form or an S form, or a racemate.

Specific examples of the amino-protecting group include, but are not limited to, the following: an acyl group such as formyl group, acetyl group, chloroacetyl group, dichloroacetyl group, trichloroacetyl group, trifluoroacetyl group, propionyl group, benzoyl group, 4-chlorobenzoyl group and the like; an optionally substituted alkoxycarbonyl group such as methoxycarbonyl group, ethoxycarbonyl group, t-butoxycarbonyl group, benzyloxycarbonyl group, allyloxycarbonyl group and the like; an optionally substituted arylalkyl group such as benzyl group, 4-methoxybenzyl group, 4-bromobenzyl group, 1-phenylethyl group, 1-(1-naphthyl)ethyl group, 1-(2-naphthyl)ethyl group, carboxyphenylmethyl group, carbamoylphenylmethyl group, 2-hydroxy-1-phenylethyl group and the like; an optionally substituted allyl group such as allyl group, crotyl group and the like; propargyl group; a sulfonyl group such as methanesulfonyl group, p-toluenesulfonyl group, 2-nitrobenzenesulfonyl group and the like. When the amino-protecting group has an asymmetric center, it may be an R form or an S form, or a racemate.

Of these, $R^2$ is preferably a hydrogen atom, an acyl group, an optionally substituted alkoxycarbonyl group, or an optionally substituted arylalkyl group, more preferably a hydrogen atom or an easily removable acetyl group, chloroacetyl group, trifluoroacetyl group, benzoyl group, t-butoxycarbonyl group, benzyloxycarbonyl group, benzyl group, 4-methoxybenzyl group, 4-bromobenzyl group, 1-phenylethyl group, 1-(1-naphthyl)ethyl group, 1-(2-naphthyl)ethyl group, or carbamoylphenylmethyl group, particularly preferably a hydrogen atom, or benzyl group, 1-phenylethyl group, or carbamoylphenylmethyl group, which is introducible by using primary amine in step (a), and industrially economical.

In the present invention, each $R^3$ is independently a hydrogen atom, an optionally substituted alkyl group, an optionally substituted aryl group, or an optionally substituted heteroaryl group, an optionally substituted hydroxyl group, an optionally substituted amino group, an optionally substituted thiol group, or a halogen atom. When possible, two or more $R^3$ may form one or plural cyclic structures.

Examples of the "alkyl group" of the "optionally substituted alkyl group" include a straight chain, branched chain or cyclic alkyl group having 1 - 10 carbon atoms, for example, a straight chain alkyl group having 1 - 10 carbon atoms such as methyl group, ethyl group, n-propyl group, n-butyl group, n-pentyl group, n-hexyl group, n-heptyl group, n-octyl group, n-nonyl group, n-decyl group and the like; a branched chain alkyl group having 3 - 10 carbon atoms such as isopropyl group, 1-methylpropyl group, t-butyl group and the like; and a cyclic alkyl group having 3 - 10 carbon atoms such as cyclopropyl group, cyclopentyl group, cyclohexyl group and the like.

Examples of the substituent that the alkyl group optionally has include a halogen atom (e.g., fluorine atom, chlorine atom, bromine atom, iodine atom); an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from an alkyl group having 1 - 6 carbon atoms (e.g., methyl group, ethyl group, isopropyl group etc.), a halogen atom (e.g., fluorine atom, chlorine atom, bromine atom, iodine atom), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

As the substituted alkyl group, benzyl group, 4-methoxybenzyl group, allyl group, 2-chloroethyl group and the like can be specifically mentioned.

Examples of the "aryl group" of the "optionally substituted aryl group" include an aromatic hydrocarbon group having 6 - 10 carbon atoms, for example, phenyl group, 1-naphthyl group, 2-naphthyl group and the like.

Examples of the substituent that the aryl group optionally has include a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from halogen atom (e.g., fluorine atom, chlorine atom, bromine atom, iodine atom), an alkyl group having 1 - 6 carbon atoms (e.g., methyl group, ethyl group, isopropyl group etc.), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

Examples of the "heteroaryl group" of the "optionally substituted heteroaryl group" include a 5- or 6-membered aromatic heterocyclic group containing, as a ring-constituting atom besides carbon atoms, 1 to 4 heteroatoms selected from a nitrogen atom, an oxygen atom and a sulfur atom, for example, pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), pyrazolyl (e.g., 1-pyrazolyl, 3-pyrazolyl, 4-pyrazolyl), imidazolyl (e.g., 1-imidazolyl, 2-imidazolyl, 4-imidazolyl), isoxazolyl (e.g., 3-isoxazolyl, 4-isoxazolyl, 5-isoxazolyl), oxazolyl (e.g., 2-oxazolyl, 4-oxazolyl, 5-oxazolyl), isothiazolyl (e.g., 3-isothiazolyl, 4-isothiazolyl, 5-isothiazolyl), thiazolyl (e.g., 2-thiazolyl, 4-thiazolyl, 5-thiazolyl), triazolyl (1,2,3-triazol-4-yl, 1,2,4-triazol-3-yl), oxadiazolyl (1,2,4-oxadiazol-3-yl, 1,2,4-oxadiazol-5-yl), thiadiazolyl (1,2,4-thiadiazol-3-yl, 1,2,4-thiadiazol-5-yl), tetrazolyl, pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl, 3-pyrazinyl) and the like.

Examples of the substituent that the heteroaryl group optionally has include a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group etc.); an alkoxy group having 1 - 6 carbon atoms (e.g., methoxy group, ethoxy group etc.); an aryl group having 6 - 10 carbon atoms (e.g., phenyl group, naphthyl group etc.) optionally having 1 to 3 substituents selected from a halogen atom (e.g., fluorine atom, chlorine atom, bromine atom, iodine atom), an alkyl group having 1 - 6 carbon atoms (e.g., methyl group, ethyl group, isopropyl group etc.), an alkenyl group having 2 - 6 carbon atoms (e.g., vinyl group etc.), an alkoxy group having 1 - 6 carbon atoms (e.g., methoxy group, ethoxy group etc.) and the like, and the like. While the number of the substituents is not particularly limited, 1 - 3 is preferable. When two or more substituents are present, the kind of the substituent may be the same or different.

As the optionally substituted hydroxyl group, a hydroxyl group and a protected form thereof; an alkoxy group having 1 - 10 carbon atoms such as methoxy group, ethoxy group, n-propoxy group, n-butoxy group, n-decyloxy group, 1-methylethoxy group, 1,1-dimethylethoxy group, cyclopropyloxy group, cyclohexyloxy group and the like; an aryloxy group having 6 - 10 carbon atoms such as phenyloxy group, 2-naphthyloxy group and the like; a heteroaryloxy group such as 2-thienyloxy group, 3-pyridyloxy group and the like can be mentioned, and the alkoxy group, aryloxy group and heteroaryloxy group may have any substituent selected from a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group, allyl group etc.); an alkoxy group having 1 - 6 carbon atoms (e.g., methoxy group, ethoxy group etc.); an arylalkyl group (e.g., benzyl group etc.) and the like. While the number of the substituents is not particularly limited, 1 - 3 is preferable. When two or more substituents are present, the kind of the substituent may be the same or different.

A protecting group used as a hydroxyl protecting group is not particularly limited as long as it can be removed under general conditions. Specific examples thereof include an acyl group such as formyl group, acetyl group, chloroacetyl group, propionyl group, benzoyl group and the like; an optionally substituted arylalkyl group such as benzyl group, 4-methoxybenzyl group, 4-bromobenzyl group, 1-phenylethyl group and the like; an acetal type protecting group such as methoxymethyl group, ethoxyethyl group, benzyloxymethyl group and the like; a silyl group such as trimethylsilyl group, t-butyldimethylsilyl group and the like.

The optionally substituted amino group optionally has any 1 or 2 substituents and/or protecting groups.

Specific examples of the substituent include, but are not limited to, the following: an alkyl group having 1 - 6 carbon atoms (e.g., methyl group, ethyl group, isopropyl group etc.); an alkenyl group having 2 - 6 carbon atoms (e.g., vinyl group, allyl group etc.); a hydroxyl group; an alkoxy group having 1 - 6 carbon atoms (e.g., methoxy group, ethoxy group etc.); and an arylalkyl group (e.g., benzyl group etc.).

Specific examples of the protecting group include, but are not limited to, the following: an acyl group such as formyl group, acetyl group, chloroacetyl group, dichloroacetyl group, trichloroacetyl group, trifluoroacetyl group, propionyl group, benzoyl group, 4-chlorobenzoyl group and the like; an optionally substituted alkoxycarbonyl group such as methoxycarbonyl group, ethoxycarbonyl group, t-butoxycarbonyl group, benzyloxycarbonyl group, allyloxycarbonyl group and the like; an optionally substituted arylalkyl group such as benzyl group, 4-methoxybenzyl group, 4-bromobenzyl group, 1-phenylethyl group and the like; and a sulfonyl group such as methanesulfonyl group, p-toluenesulfonyl group, 2-nitrobenzenesulfonyl group and the like.

As the optionally substituted thiol group, a thiol group and a protected form thereof; an alkylthio group having 1 - 10 carbon atoms such as methylthio group, ethylthio group, n-propylthio group, n-butylthio group, n-decylthio group, 1-methylethylthio group, 1,1-dimethylethylthio group, cyclopropylthio group, cyclohexylthio group and the like; an arylthio group having 6 - 10 carbon atoms such as phenylthio group, 2-naphthylthio group and the like; a heteroarylthio group such as 2-thienylthio group, 3-pyridylthio group and the like can be mentioned, and the alkylthio group, arylthio group and heteroarylthio group may have any substituent selected from a halogen atom (e.g., fluorine atom, chlorine atom, bromine atom, iodine atom); an alkyl group having 1 - 6 carbon atoms (e.g., methyl group, ethyl group, isopropyl group etc.); an alkenyl group having 2 - 6 carbon atoms (e.g., vinyl group, allyl group etc.); an alkoxy group having 1 - 6 carbon atoms (e.g., methoxy group, ethoxy group etc.); an arylalkyl group (e.g., benzyl group etc.) and the like. While the number

of the substituents is not particularly limited, 1 - 3 is preferable. When two or more substituents are present, the kind of the substituent may be the same or different.

A protecting group used as a thiol protecting group is not particularly limited as long as it can be removed under general conditions. Specific examples thereof include an acyl group such as formyl group, acetyl group, chloroacetyl group, propionyl group, benzoyl group and the like; an optionally substituted arylalkyl group such as benzyl group, 4-methoxybenzyl group, 4-bromobenzyl group, 1-phenylethyl group and the like; an acetal type protecting group such as methoxymethyl group, ethoxyethyl group, benzyloxymethyl group and the like; and a silyl group such as trimethylsilyl group, t-butyldimethylsilyl group and the like.

Specific examples of the halogen atom are a fluorine atom, a chlorine atom, a bromine atom, and an iodine atom.

Of these, $R^3$ is preferably a hydrogen atom.

In the present invention, X is a halogen atom, or a sulfonyloxy group.

Specific examples of the halogen atom are a fluorine atom, a chlorine atom, a bromine atom, and an iodine atom.

Specific examples of the sulfonyloxy group include, but are not limited to, the following: an optionally substituted alkylsulfonyloxy group such as methanesulfonyloxy group, chloromethanesulfonyloxy group, trifluoromethanesulfonyloxy group and the like; an optionally substituted arylsulfonyloxy group such as p-toluenesulfonyloxy group, p-chlorobenzenesulfonyloxy group, 2-nitrobenzenesulfonyloxy group and the like.

Of these, X is preferably a halogen atom, more preferably a chlorine atom corresponding to an industrially economical acyclic ketone compound.

In the present invention, Y is a nitrogen atom or a nitrogen atom substituted by $R^2$.

In the present invention, Z is a carbon atom or a carbon atom substituted by a cyano group.

The acyclic ketone compound represented by the above-mentioned formula (1) is a compound having a leaving group such as a halogen atom and the like at the 4-position of 1-substituted-1-butanone.

Specific examples of the acyclic ketone compound represented by the above-mentioned formula (1) include 5-fluoro-2-pentanone, 5-chloro-2-pentanone, 5-bromo-2-pentanone, 5-iodo-2-pentanone, 5-(methanesulfonyloxy)-2-pentanone, 5-(chloromethanesulfonyloxy)-2-pentanone, 5-(toluenesulfonyloxy)-2-pentanone, 6-chloro-3-hexanone, 6-bromo-3-hexanone, 1-chloro-4-octanone, 6-chloro-2-methyl-3-hexanone, 4-chloro-1-cyclopropyl-1-butanone, 4-chloro-1-cyclohexyl-1-butanone, 4-bromo-1-cyclohexyl-1-butanone, 7-chloro-1-hepten-4-one, 4-chloro-1-phenyl-1-butanone, 4-bromo-1-phenyl-1-butanone, 4-chloro-1-(4-methoxyphenyl)-1-butanone, 4-chloro-1-(4-chlorophenyl)-1-butanone, 4-chloro-1-(2-thienyl)-1-butanone, and 4-chloro-1-(3-pyridyl)-1-butanone.

The cyclic nitrogen-containing compound represented by the above-mentioned formula (2) is a pyrrolidine which is a saturated 5-membered ring amine, or a pyrroline which is an unsaturated 5-membered ring imine having a carbon-nitrogen double bond, and has a substituent at the 2-position.

When the cyclic nitrogen-containing compound represented by the above-mentioned formula (2) is a pyrrolidine, the compound is particularly a 2-cyanopyrrolidine represented by the following formula (7), i.e., a compound having substituent $R^1$ and a cyano group at the 2-position of pyrrolidine, and substituent $R^2$ on the nitrogen atom of pyrrolidine.

The 2-cyanopyrrolidine represented by the following formula (7) has an asymmetric center at the carbon atom to which the cyano group is bonded. When an asymmetric center other than this is absent in a molecule, it is generally a racemate. When plural asymmetric centers are present in a molecule, it is generally a diastereomeric mixture.

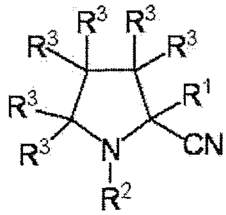
(7)

Specific examples of 2-cyanopyrrolidines represented by the above-mentioned formula (7) include 2-cyano-2-methylpyrrolidine, 1-acetyl-2-cyano-2-methylpyrrolidine, 1-(t-butoxycarbonyl)-2-cyano-2-methylpyrrolidine, 1-(chloroacetyl)-2-cyano-2-methylpyrrolidine, 2-cyano-2-methyl-1-(trifluoroacetyl)pyrrolidine, 1-benzoyl-2-cyano-2-methylpyrrolidine, 1-(benzyloxycarbonyl)-2-cyano-2-methylpyrrolidine, 1-benzyl-2-cyano-2-methylpyrrolidine, 1-(1-phenylethyl)-2-cyano-2-methylpyrrolidine, 1-(1-(1-naphthyl)ethyl)-2-cyano-2-methylpyrrolidine, 1-(1-(2-naphthyl)ethyl)-2-cyano-2-methylpyrrolidine, 1-(carbamoylphenylmethyl)-2-cyano-2-methylpyrrolidine, 2-cyano-2-ethylpyrrolidine, 2-butyl-2-cyanopyrrolidine, 1-(t-butoxycarbonyl)-2-butyl-2-cyanopyrrolidine, 2-cyano-2-(1-methylethyl)pyrrolidine, 2-cyano-2-cyclopropylpyrrolidine, 2-cyano-2-cyclohexylpyrrolidine, 2-allyl-2-cyanopyrrolidine, 2-cyano-2-phenylpyrrolidine, 1-(t-butoxycarbonyl)-2-cyano-2-phenylpyrrolidine, 1-benzyl-2-cyano-2-phenylpyrrolidine, 1-benzyl-2-cyano-2-(4-methoxyphenyl)pyrrolidine, 1-benzyl-2-(4-chlorophenyl)-2-cyanopyrrolidine, 1-benzyl-2-cyano-2-(2-thienyl)pyrrolidine, and 1-benzyl-2-cyano-2-(3-pyridyl)pyrrolidine.

When the cyclic nitrogen-containing compound represented by the above-mentioned formula (2) is a pyrroline, the compound is particularly a pyrroline represented by the following formula (6), i.e., a compound having substituent $R^1$ at the 2-position of 1-pyrroline.

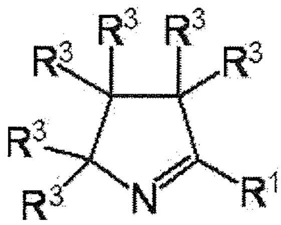

(6)

Specific examples of pyrrolines represented by the above-mentioned formula (6) include 2-methyl-1-pyrroline, 2-ethyl-1-pyrroline, 2-butyl-1-pyrroline, 2-(1-methylethyl)-1-pyrroline, 2-cyclopropyl-1-pyrroline, 2-cyclohexyl-1-pyrroline, 2-allyl-1-pyrroline, 2-phenyl-1-pyrroline, 2-(4-methoxyphenyl)-1-pyrroline, 2-(4-chlorophenyl)-1-pyrroline, 2-(2-thienyl)-1-pyrroline, and 2-(3-pyridyl)-1-pyrroline.

The α-substituted prolinamide represented by the above-mentioned formula (3) is a compound having a substituent and a carbamoyl group at the 2-position of pyrrolidine, and a hydrogen atom, an optionally substituted alkyl group, or an amino-protecting group on the nitrogen atom of pyrrolidine.

The α-substituted prolinamide represented by the above-mentioned formula (3) has an asymmetric center on the carbon atom to which the carbamoyl group is bonded.

When an asymmetric center is absent except at the carbon atom to which the carbamoyl group is bonded, it may be a racemate or an optically active form of any of S form and R form having any optical purity of 0 - 99%ee. In this case, the optical purity is preferably not more than 80%ee, more preferably not more than 60%ee, particularly preferably not more than 50%ee.

When plural asymmetric centers are present in a molecule, it may be a diastereomer or a diastereomeric mixture of any stereochemistry. In this case, the diastereomeric purity may be any, and it is generally a diastereomer or a diastereomeric mixture having any ratio of R form:S form (molar ratio)=0.5:99.5 - 99.5:0.5 in the stereochemistry of the 2-position of pyrrolidine. Preferably, the stereochemistry of the 2-position is R form:S form (molar ratio)=10:90 - 90:10, more preferably R form:S form (molar ratio)=20:80 - 80:20, particularly preferably R form:S form (molar ratio)=25:75-75:25. Moreover, a mixture having a lower ratio of R form to S form is preferable since it can be synthesized more easily.

Specific examples of the α-substituted prolinamides represented by the above-mentioned formula (3) include α-methylprolinamide, N-acetyl-α-methylprolinamide, N-(t-butoxycarbonyl)-α-methylprolinamide, N-(chloroacetyl)-α-methylprolinamide, N-(trifluoroacetyl)-α-methylprolinamide, N-benzoyl-α-methylprolinamide, N-(benzyloxycarbonyl)-α-methylprolinamide, N-benzyl-α-methylprolinamide, N-(1-phenylethyl)-α-methylprolinamide, N-(1-(1-naphthyl)ethyl)-α-methylprolinamide, N-(1-(2-naphthyl)ethyl)-α-methylprolinamide, N-(carbamoylphenylmethyl)-α-methylprolinamide, α-ethylprolinamide, α-butylprolinamide, N-(t-butoxycarbonyl)-α-butylprolinamide, α-(1-methylethyl)prolinamide, α-cyclopropylprolinamide, α-cyclohexylprolinamide, α-allylprolinamide, α-phenylprolinamide, N-(t-butoxycarbonyl)-α-phenylprolinamide, N-benzyl-α-phenylprolinamide, α-(4-methoxyphenyl)prolinamide, N-benzyl-α-(4-methoxyphenyl)prolinamide, α-(4-chlorophenyl)prolinamide, N-benzyl-α-(4-chlorophenyl)prolinamide, α-(2-thienyl)prolinamide, N-benzyl-α-(2-thienyl)prolinamide, α-(3-pyridyl)prolinamide, and N-benzyl-α-(3-pyridyl)prolinamide.

The optionally active α-substituted proline represented by the above-mentioned formula (4) is a compound having a substituent and a carboxyl group at the 2-position of pyrrolidine, and a hydrogen atom, an optionally substituted alkyl group, or an amino-protecting group on the nitrogen atom of pyrrolidine. The optionally active α-substituted proline represented by the above-mentioned formula (4) is an optionally active form having an asymmetric center on the carbon atom to which the carboxyl group is bonded. When an asymmetric center is absent other than this in a molecule, it may be any of S form and R form. While the optical purity may be any, it is preferably not less than 80%ee, more preferably not less than 90%ee, further preferably not less than 95%ee. Since pharmaceutical products and intermediates therefor require high optical purity, it is particularly preferably not less than 99%ee.

When plural asymmetric centers are present in a molecule, it may be a diastereomer or a diastereomeric mixture having any stereochemistry. While the diastereomeric purity and/or optical purity may be any, the stereochemistry of the 2-position of pyrrolidine is preferably R form:S form (molar ratio)=90:10 - 100:0 or R form:S form (molar ratio)=10:90-0:100, more preferably R form:S form (molar ratio)=97.5:2.5-100:0 or R form:S form (molar ratio)=2.5:97.5 - 0:100. Since pharmaceutical products and intermediates therefor require high optical purity, it is more preferably R form:S form (molar ratio)=99:1 - 100:0 or R form:S form (molar ratio)=1:99 - 0:100, particularly preferably R form:S form (molar ratio)=99.5:0.5-100:0 or R form:S form (molar ratio)=0.5:99.5 - 0:100.

Specific examples of the optically active α-substituted proline represented by the above-mentioned formula (4) include (R)-α-methylproline, (R)-N-acetyl-α-methylproline, (R)-N-(t-butoxycarbonyl)-α-methylproline, (R)-N-(chloroacetyl)-α-methylproline, (R)-N-(trifluoroacetyl)-α-methylproline, (R)-N-benzoyl-α-methylproline, (R)-N-(benzyloxycarbonyl)-α-methylproline, (R)-N-benzyl-α-methylproline, (R)-α-ethylproline, (R)-α-butylproline, (R)-N-(t-butoxycarbonyl)-α-butylproline, (R)-α-(1-methylethyl)proline, (R)-α-cyclopropylproline, (R)-α-cyclohexylproline, (R)-α-allylproline, (R)-α-phenylproline, (R)-N-(t-butoxycarbonyl)-α-phenylproline, (R)-N-benzyl-α-phenylproline, (R)-α-(4-methoxyphenyl)proline, (R)-α-(4-chlorophenyl)proline, (R)-α-(2-thienyl)proline, (R)-α-(3-pyridyl)proline, (S)-α-methylproline, (S)-N-acetyl-α-methylproline, (S)-N-(t-butoxycarbonyl)-α-methylproline, (S)-N-(chloroacetyl)-α-methylproline, (S)-N-(trifluoroacetyl)-α-methylproline, (S)-N-benzoyl-α-methylproline, (S)-N-(benzyloxycarbonyl)-α-methylproline, (S)-N-benzyl-α-methylproline, (S)-α-ethylproline, (S)-α-butylproline, (S)-N-(t-butoxycarbonyl)-α-butylproline, (S)-α-(1-methylethyl)proline, (S)-α-cyclopropylproline, (S)-α-cyclohexylproline, (S)-α-allylproline, (S)-α-phenylproline, (S)-N-(t-butoxycarbonyl)-α-phenylproline, (S)-N-benzyl-α-phenylproline, (S)-α-(4-methoxyphenyl)proline, (S)-α-(4-chlorophenyl)proline, (S)-α-(2-thienyl)proline, (S)-α-(3-pyridyl)proline, (2R,1'R)-N-(1'-phenylethyl)-α-methylproline, (2R,1'S)-N-(1'-phenylethyl)-α-methylproline, (2S,1'R)-N-(1'-phenylethyl)-a-methylproline, (2S,1'S)-N-(1'-phenylethyl)-α-methylproline, (2R,1'R)-N-(1'-(1-naphthyl)ethyl)-α-methylproline, (2R,1'S)-N-(1'-(1-naphthyl)ethyl)-α-methylproline, (2S,1'R)-N-(1'-(1-naphthyl)ethyl)-α-methylproline, (2S,1'S)-N-(1'-(1-naphthyl)ethyl)-α-methylproline, (2R,1'R)-N-(1'-(2-naphthyl)ethyl)-α-methylproline, (2R,1'S)-N-(1'-(2-naphthyl)ethyl)-α-methylproline, (2S,1'R)-N-(1'-(2-naphthyl)ethyl)-α-methylproline, (2S,1'S)-N-(1'-(2-naphthyl)ethyl)-α-methylproline, (2R,1'R)-N-(carbamoylphenylmethyl)-α-methylproline, (2R,1'S)-N-(carbamoylphenylmethyl)-α-methylproline, (2S,1'R)-N-(carbamoylphenylmethyl)-α-methylproline, and (2S,1'S)-N-(carbamoylphenylmethyl)-α-methylproline.

The optically active α-substituted prolinamide represented by the above-mentioned formula (5) is a compound having a substituent and a carbamoyl group at the 2-position of pyrrolidine, and a hydrogen atom, an optionally substituted alkyl group, or an amino-protecting group on the nitrogen atom of pyrrolidine.

The optionally active α-substituted prolinamide represented by the above-mentioned formula (5) is an optionally active form having an asymmetric center on the carbon atom to which the carbamoyl group is bonded. When an asymmetric center is absent other than this in a molecule, it may be any of S form and R form. While the optical purity may be any, it is higher than that of the α-substituted prolinamide represented by the above-mentioned formula (3), preferably not less than 80%ee, more preferably not less than 90%ee, further preferably not less than 95%ee. Since pharmaceutical products and intermediates therefor require high optical purity, it is particularly preferably not less than 99%ee.

When plural asymmetric centers are present in a molecule, it may be a diastereomer or a diastereomeric mixture of any stereochemistry. While the diastereomeric purity may be any, the stereochemistry of the 2-position of pyrrolidine is higher than that of the α-substituted prolinamide represented by the above-mentioned formula (3), and is preferably R form:S form (molar ratio)=90:10 - 100:0 or R form:S form (molar ratio)=10:90 - 0:100, more preferably R form:S form (molar ratio)=97.5:2.5 - 100:0 or R form:S form (molar ratio)=2.5:97.5 - 0:100. Since pharmaceutical products and intermediates therefor require high optical purity, it is more preferably R form:S form (molar ratio)=99:1 - 100:0 or R form:S form (molar ratio)=1:99 - 0:100, particularly preferably R form:S form (molar ratio)=99.5:0.5 - 100:0 or R form:S form (molar ratio)=0.5:99.5 - 0:100.

Specific examples of the optically active α-substituted prolinamide represented by the above-mentioned formula (5) include (R)-α-methylprolinamide, (R)-N-acetyl-α-methylprolinamide, (R)-N-(t-butoxycarbonyl)-α-methylprolinamide, (R)-N-(chloroacetyl)-α-methylprolinamide, (R)-N-(trifluoroacetyl)-α-methylprolinamide, (R)-N-benzoyl-α-methylprolinamide, (R)-N-(benzyloxycarbonyl)-α-methylprolinamide, (R)-N-benzyl-α-methylprolinamide, (R)-α-ethylprolinamide, (R)-α-butylprolinamide, (R)-N-(t-butoxycarbonyl)-α-butylprolinamide, (R)-α-(1-methylethyl)prolinamide, (R)-α-cyclopropylprolinamide, (R)-α-cyclohexylprolinamide, (R)-α-allylprolinamide, (R)-α-phenylprolinamide, (R)-N-(t-butoxycarbonyl)-α-phenylprolinamide, (R)-N-benzyl-α-phenylprolinamide, (R)-α-(4-methoxyphenyl)prolinamide, (R)-α-(4-chlorophenyl)prolinamide, (R)-α-(2-thienyl)prolinamide, (R)-α-(3-pyridyl)prolinamide, (S)-α-methylprolinamide, (S)-N-acetyl-α-methylprolinamide, (S)-N-(t-butoxycarbonyl)-α-methylprolinamide, (S)-N-(chloroacetyl)-α-methylprolinamide, (S)-N-(trifluoroacetyl)-α-methylprolinamide, (S)-N-benzoyl-α-methylprolinamide, (S)-N-(benzyloxycarbonyl)-α-methylprolinamide, (S)-N-benzyl-α-methylprolinamide, (S)-α-ethylprolinamide, (S)-α-butylprolinamide, (S)-N-(t-butoxycarbonyl)-α-butylprolinamide, (S)-α-(1-methylethyl)prolinamide, (S)-α-cyclopropylprolinamide, (S)-α-cyclohexylprolinamide, (S)-α-allylprolinamide, (S)-α-phenylprolinamide, (S)-N-(t-butoxycarbonyl)-α-phenylprolinamide, (S)-N-benzyl-α-phenylprolinamide, (S)-α-(4-methoxyphenyl)prolinamide, (S)-α-(4-chlorophenyl)prolinamide, (S)-α-(2-thienyl)prolinamide, (S)-α-(3-pyridyl)prolinamide, (2R,1'R)-N-(1'-phenylethyl)-a-methylprolinamide, (2R,1'S)-N-(1'-phenylethyl)-α-methylprolinamide, (2S,1'R)-N-(1'-phenylethyl)-α-methylprolinamide, (2S,1'S)-N-(1'-phenylethyl)-α-methylprolinamide, (2R,1'R)-N-(1'-(1-naphthyl)ethyl)-α-methylprolinamide, (2R,1'S)-N-(1'-(1-naphthyl)ethyl)-α-methylprolinamide, (2S,1'R)-N-(1'-(1-naphthyl)ethyl)-α-methylprolinamide, (2S,1'S)-N-(1'-(1-naphthyl)ethyl)-α-methylprolinamide, (2R,1'R)-N-(1'-(2-naphthyl)ethyl)-α-methylprolinamide, (2R,1'S)-N-(1'-(2-naphthyl)ethyl)-α-methylprolinamide, (2S,1'R)-N-(1'-(2-naphthyl)ethyl)-α-methylprolinamide, (2S,1'S)-N-(1'-(2-naphthyl)ethyl)-α-methylprolinamide, (2R,1'R)-N-(carbamoylphenylmethyl)-α-methylprolinamide, (2R,1'S)-N-(carbamoylphenylmethyl)-α-methylprolinamide,

(2S,1'R)-N-(carbamoylphenylmethyl)-α-methylprolinamide, and (2S,1'S)-N-(carbamoylphenylmethyl)-α-methylprolinamide.

The above-mentioned compounds may have a basic or acidic functional group, and optionally form a salt. Examples of such salt include inorganic acid salts (e.g., hydrochloride, sulfate, nitrate, phosphate etc.); organic acid salts (e.g., acetate, propionate, methanesulfonate, 4-toluenesulfonate, oxalate, maleate etc.); tartaric acids (L-tartaric acid, D-tartaric acid, (2S,3S)-dibenzoyltartaric acid, (2R,3R)-dibenzoyltartaric acid, (2S,3S)-di(p-toluoyl)tartaric acid, (2R,3R)-di(p-toluoyl)tartaric acid etc.); mandelic acids ((S)-mandelic acid, (R)-mandelic acid etc.); amino acid derivatives (N-acetyl-L-alanine, N-acetyl-L-phenylglycine, N-acetyl-D-phenylglycine, N-benzyl-L-phenylglycine, N-benzyl-D-phenylglycine, N-acetyl-L-phenylalanine, N-acetyl-L-glutamic acid, N-acetyl-L-aspartic acid etc.); optically active sulfonic acids ((S)-10-camphorsulfonic acid, (R)-10-camphorsulfonic acid, (S)-1-phenylethanesulfonic acid, (R)-1-phenylethanesulfonic acid etc.); alkali metal salts (e.g., sodium salt, potassium salt etc.); alkaline earth metal salts (e.g., calcium salt, magnesium salt etc.); and salts with organic base (e.g., trimethylamine salt, triethylamine salt, pyridine salt, picoline salt, dicyclohexylamine salt etc.) and the like.

The α-methylprolinamide represented by the above-mentioned formula (8) is a compound having methyl group and carbamoyl group at the 2-position of pyrrolidine, and 1-phenylethyl group, 1-(1-naphthyl)ethyl group, 1-(2-naphthyl)ethyl group, or carbamoylphenylmethyl group on the nitrogen atom of pyrrolidine. The substituent on the nitrogen atom of pyrrolidine is preferably 1-phenylethyl group or carbamoylphenylmethyl group, more preferably 1-phenylethyl group.

The α-methylprolinamide has two asymmetric centers at the 2-position of pyrrolidine and the substituent (1'-position) on the nitrogen atom of pyrrolidine, and may be a diastereomer or a diastereomeric mixture of any stereochemistry. Since the asymmetric carbon at the substituent (1'-position) on the pyrrolidine nitrogen atom is derived from the primary amine or a salt thereof used in step (a), when the reaction is free of epimerization, the optical purity of the primary amine used is the asymmetric purity at the 1'-position. The absolute configuration at the 1'-position may be any of R form, S form, and racemate, and the asymmetric purity thereof may be any. However, since the isomer at the 2-position of pyrrolidine can be resolved by the asymmetry at the 1'-position, it is preferably as high as possible, and is R form:S form (molar ratio)=90:10 - 100:0 or R form:S form (molar ratio)=10:90-0:100, more preferably R form:S form (molar ratio)=97.5:2.5-100:0 or R form:S form (molar ratio)=2.5:97.5 - 0:100, particularly preferably R form:S form (molar ratio)=99.5:0.5-100:0 or R form:S form (molar ratio)=0.5:99.5 - 0:100, for the 1'-position.

Since the α-methylprolinamide represented by the above-mentioned formula (8) may form a salt with an optically active acid and/or achiral acid, and forms diastereomeric salts permitting resolution, it is useful. Specific examples of such salt include inorganic acid salts (e.g., hydrochloride, sulfate, nitrate, phosphate etc.); organic acid salts (e.g., acetate, propionate, methanesulfonate, 4-toluenesulfonate, oxalate, maleate etc.); tartaric acids (L-tartaric acid, D-tartaric acid, (2S,3S)-dibenzoyltartaric acid, (2R,3R)-dibenzoyltartaric acid, (2S,3S)-di(p-toluoyl)tartaric acid, (2R,3R)-di(p-toluoyl)tartaric acid etc.); mandelic acids ((S)-mandelic acid, (R)-mandelic acid etc.); amino acid derivatives (N-acetyl-L-alanine, N-acetyl-L-phenylglycine, N-acetyl-D-phenylglycine, N-benzyl-L-phenylglycine, N-benzyl-D-phenylglycine, N-acetyl-L-phenylalanine, N-acetyl-L-glutamic acid, N-acetyl-L-aspartic acid etc.); optically active sulfonic acids ((S)-10-camphorsulfonic acid, (R)-10-camphorsulfonic acid, (S)-1-phenylethanesulfonic acid, (R)-1-phenylethanesulfonic acid etc.) and the like. Of these, preferred are salts with optically active acid expected to show a large difference in the solubility between diastereomeric salts, more preferred are salts with tartaric acids, mandelic acids, and particularly preferred are salts with L-tartaric acid, D-tartaric acid, (S)-mandelic acid, (R)-mandelic acid, which are economical.

Specific examples of the salt of N-(1'-phenylethyl)-α-methylprolinamide with optically active acid and/or achiral acid include (2S,1'S)-N-(1'-phenylethyl)-α-methylprolinamide D-tartrate, (2S,1'S)-N-(1'-phenylethyl)-α-methylprolinamide L-tartrate, (2S,1'R)-N-(1'-phenylethyl)-α-methylprolinamide D-tartrate, (2S,1'R)-N-(1'-phenylethyl)-α-methylprolinamide L-tartrate, (2R,1'R)-N-(1'-phenylethyl)-α-methylprolinamide D-tartrate, (2R,1'R)-N-(1'-phenylethyl)-α-methylprolinamide L-tartrate, (2R,1'S)-N-(1'-phenylethyl)-α-methylprolinamide D-tartrate, (2R,1'S)-N-(1'-phenylethyl)-α-methylprolinamide L-tartrate, (2S,1'S)-N-(1'-phenylethyl)-α-methylprolinamide (S)-mandelate, (2S,1'S)-N-(1'-phenylethyl)-α-methylprolinamide (R)-mandelate, (2S,1'R)-N-(1'-phenylethyl)-α-methylprolinamide (S)-mandelate, (2S,1'R)-N-(1'-phenylethyl)-α-methylprolinamide (R)-mandelate, (2R,1'R)-N-(1'-phenylethyl)-α-methylprolinamide (S)-mandelate, (2R,1'R)-N-(1'-phenylethyl)-α-methylprolinamide (R)-mandelate, (2R,1'S)-N-(1'-phenylethyl)-α-methylprolinamide (S)-mandelate, (2R,1'S)-N-(1'-phenylethyl)-α-methylprolinamide (R)-mandelate, (2S,1'S)-N-(1'-phenylethyl)-α-methylprolinamide 4-toluenesulfonate, (2R,1'R)-N-(1'-phenylethyl)-α-methylprolinamide 4-toluenesulfonate, (2S,1'S)-N-(1'-phenylethyl)-α-methylprolinamide oxalate, and (2R,1'R)-N-(1'-phenylethyl)-α-methylprolinamide oxalate.

Step (a)

The cyclic nitrogen-containing compound represented by the above-mentioned formula (2) can be synthesized by reacting an acyclic ketone compound represented by the above-mentioned formula (1) with at least one selected from ammonia, an ammonium salt, primary amine and a salt of primary amine, and a cyanating agent.

The acyclic ketone compound represented by the above-mentioned formula (1) can be purchased as reagents such as 5-chloro-2-pentanone, 4-chloro-1-phenyl-1-butanone and the like. Other compounds can be freely produced by a method such as Friedel-Crafts reaction of 3-chloropropionyl chloride and an aromatic compound, Claisen condensation of γ-butyrolactone and esters, followed by a treatment with hydrogen halide and the like (see, for example, Chem. Pharm. Bull., 1989, 37, 958).

The at least one selected from ammonia, an ammonium salt, primary amine and a salt of primary amine used in step (a) only needs to be a compound capable of providing ammonia or primary amine in the reaction system. Specific examples thereof include, but are not limited to, the following: ammonia; primary amine such as benzylamine, 4-methoxybenzylamine, 4-bromobenzylamine, α-methylbenzylamine, 1-(1-naphthyl)ethylamine, α-phenylglycine, α-phenylglycinamide, α-phenylglycinol, allylamine, propargylamine and the like; and salts thereof. As the salt of ammonia, namely, ammonium salt, ammonium salt with mineral acid such as ammonium chloride, ammonium sulfate, ammonium hydrogen sulfate, ammonium nitrate and the like; ammonium salt with inorganic acid such as ammonium carbonate, ammonium hydrogen carbonate, monoammonium phosphate, diammonium hydrogen phosphate and the like; ammonium salt with organic acid such as ammonium acetate, ammonium formate, ammonium citrate and the like can be specifically mentioned. A mixture of two or more kinds selected from these ammonia, ammonium salts, primary amine and salts of primary amine may be used. When ammonia is used as at least one selected from ammonia, an ammonium salt, primary amine and a salt of primary amine, aqueous ammonia, ammonia methanol solution, ammonia gas and the like can be used. When primary amine is used as at least one selected from ammonia, an ammonium salt, primary amine and a salt of primary amine, a salt such as hydrochloride, acetate, carbonate and the like can be used and, when it contains an asymmetric center, it may be an R form or S form, or a racemate. Use of primary amine and/or a salt thereof as at least one selected from ammonia, an ammonium salt, primary amine and a salt of primary amine is preferable since the 2-cyanopyrrolidines represented by the above-mentioned formula (7) can be obtained without by producing pyrroline represented by the above-mentioned formula (6). Of these, preferred are economical benzylamine, and (S)-α-methylbenzylamine and (R)-α-methylbenzylamine capable of diastereoselective reaction due to the asymmetric center they have. On the other hand, use of ammonia and/or a salt thereof as at least one selected from ammonia, an ammonium salt, primary amine and a salt of primary amine is preferable since a deprotection step is not necessary for the production of optically active α-substituted prolines, thus affording a fewer steps. Of these, preferred are ammonium chloride, ammonium acetate, ammonium formate, and aqueous ammonia, which are industrially inexpensive and sufficiently dissolve in reaction solvents, more preferred are ammonium acetate and ammonium formate having buffering capacity and capable of controlling the reaction solution at near neutral.

The amount of at least one selected from ammonia, an ammonium salt, primary amine and a salt of primary amine to be used is 0.5 - 10 equivalents, preferably 0.8 - 5 equivalents, more preferably 0.9 - 3 equivalents, relative to the acyclic ketone compound represented by the above-mentioned formula (1).

Specific examples of the cyanating agent used in step (a) include, but are not limited to, the following: inorganic cyanide such as sodium cyanide, potassium cyanide, copper cyanide and the like; organic cyanide such as trimethylsilyl cyanide, tetrabutylammonium cyanide, tributyltin cyanide and the like; cyanohydrins such as hydrocyanic acid; acetone cyanohydrin and the like; aminonitrile compounds such as 2-amino-2-methylpropanenitrile and the like, and the like. Plural cyanating agents selected therefrom may be used in a mixture. When the cyanating agent to be used is an aminonitrile compound, it may also act as at least one selected from ammonia, an ammonium salt, primary amine and a salt of primary amine. Of these, preferred is spontaneously decomposable inorganic cyanide with a low risk of generating an extremely poisonous hydrocyanic acid gas, more preferred are industrially economical sodium cyanide and potassium cyanide.

Excess use of a cyanating agent is not preferable since a high concentration cyanide waste liquid is produced. For the production of the 2-cyanopyrrolidine represented by the above-mentioned formula (7), the amount of a cyanating agent to be used is 1 - 3 equivalents, preferably 1.0 - 1.5 equivalents, more preferably 1.0 - 1.2 equivalents, relative to the acyclic ketone compound represented by the above-mentioned formula (1). For the production of the pyrroline represented by the above-mentioned formula (6), moreover, since the pyrroline represented by the above-mentioned formula (6), which is the resultant product, does not contain a cyano group, the amount of a cyanating agent to be used may be a catalytic amount, and 0.1 - 3 equivalents, preferably 0.2 - 1.0 equivalent, more preferably 0.2 - 0.5 equivalent, relative to the acyclic ketone compound represented by the above-mentioned formula (1).

In step (a), it is preferable to add an acidic substance to make the inside of the reaction system weakly acidic or weakly alkaline. An acidic substance is added to suppress side reactions that may occur in strong alkalinity such as cyclopropanation and the like, and to carry out the reaction smoothly. Therefore, it may be a compound that develops acidity by hydrolysis and the like in the reaction system even though it is not acidic when added, such as carboxylic acid ester and the like, or a salt of a weak base with an acid such as ammonium salt and the like. Specific examples thereof include, but are not limited to, the following: mineral acids such as hydrochloric acid, sulfuric acid, nitric acid and the like; carboxylic acids such as acetic acid, formic acid, trifluoroacetic acid, benzoic acid, oxalic acid and the like; sulfonic acids such as methanesulfonic acid, toluenesulfonic acid and the like; phosphoric acids such as phosphoric acid, sodium

dihydrogen phosphate, potassium dihydrogen phosphate, disodium hydrogen phosphate and the like; carboxylic acid esters such as ethyl acetate, methyl acetate, methyl benzoate and the like; ammonium salts with mineral acid such as ammonium chloride, ammonium sulfate, ammonium hydrogen sulfate, ammonium nitrate and the like; ammonium salts with inorganic acid such as ammonium carbonate, ammonium hydrogen carbonate, monoammonium phosphate, diammonium hydrogen phosphate and the like; and ammonium salts with organic acid such as ammonium acetate, ammonium formate, ammonium citrate and the like. Plural acidic substances selected therefrom may be used in a mixture.

When an ammonium salt is used as at least one selected from ammonia, an ammonium salt, primary amine and a salt of primary amine in step (a), it is preferable to use the ammonium salt also as an acidic substance since the kind of the substance to be added can be reduced and the reaction system can be more simplified. More preferred are industrially economical ammonium chloride, ammonium acetate and ammonium formate, which dissolve sufficiently in a reaction solvent, particularly preferred are ammonium acetate and ammonium formate. When a substance other than an ammonium salt is used as at least one selected from ammonia, an ammonium salt, primary amine and a salt of primary amine in step (a), preferred from among these acidic substances are weakly acidic carboxylic acids and carboxylic acid esters capable of maintaining the reaction mixture weakly acidic even when hydrogen chloride is developed due to the reaction, or phosphoric acids showing high buffering capacity, more preferred are carboxylic acids, particularly preferred are industrially economical acetic acid and formic acid.

The amount of the acidic substance to be used only needs to be an amount capable of partial neutralization preventing strong alkalinity, and is 0.01 - 10 equivalents, preferably 0.1 - 5 equivalents, more preferably 0.3 - 3 equivalents, relative to the acyclic ketone compound represented by the above-mentioned formula (1).

While the solvent to be used in step (a) is not particularly limited as long as it does not adversely influence the reaction, hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as acetone, methyl ethyl ketone, methyl isobutyl ketone and the like; amide solvents such as dimethylformamide, N-methylpyrrolidone and the like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, t-butanol and the like; water and the like can be specifically mentioned. Plural solvents selected therefrom may be used in a mixture at any ratio. Examples of preferable solvent include tetrahydrofuran, acetone, dimethylformamide, acetonitrile, dimethyl sulfoxide, alcohol solvents, water, and a mixed solvent containing these, which dissolve the acyclic ketone compound represented by the above-mentioned formula (1), at least one selected from ammonia, an ammonium salt, primary amine and a salt of primary amine, and a cyanating agent. More preferred are alcohol solvents, water, and a mixed solvent containing these, which dissolve sodium cyanide and potassium cyanide, which are preferable cyanating agents, an acidic substance preferably added and the like, comparatively well and are capable of suppressing side reactions by setting the inside of the reaction system weakly acidic or weakly alkaline. Particularly preferred are water and a mixed solvent containing water. As the amount of the solvent to be used, any amount of the solvent can be used. Generally, it is 1- to 50-fold volume, preferably 1- to 20-fold volume, more preferably 2- to 10-fold volume, relative to the acyclic ketone compound represented by the above-mentioned formula (1).

While the reaction temperature of step (a) is not particularly limited as long as the reaction is not adversely influenced, it is generally -20 - 120°C, preferably 10 - 80°C, more preferably 30 - 70°C.

While the reaction time of step (a) is not particularly limited as long as the reaction is not adversely influenced, it is preferably 10 min - 24 hr, more preferably 1 - 10 hr, to suppress the production cost.

The cyclic nitrogen-containing compound represented by the above-mentioned formula (2) obtained step (a) can also be purified by a method such as extraction and/or distillation and the like, or can also be used for the next step without purification.

Particularly, when the cyclic nitrogen-containing compound represented by the above-mentioned formula (2) is the 2-cyanopyrrolidine represented by the above-mentioned formula (7), sufficient purity and water removal can be achieved by extraction alone. Therefore, it is preferable to use the obtained compound for the next step after a concentration operation as necessary, without a further purification operation, since it simplifies the operation and increases the producibility. In addition, the nitrogen atom on the pyrrolidine ring may be protected before using for step (b).

On the other hand, when the cyclic nitrogen-containing compound represented by the above-mentioned formula (2) is the pyrroline represented by the above-mentioned formula (6), since it is generally a low boiling point compound, when purification is necessary, it is preferably purified by distillation and, when purification is not necessary, it is preferably used without purification for other applications. The pyrroline represented by the above-mentioned formula (6) can be converted to the 2-cyanopyrrolidine represented by the above-mentioned formula (7) by reacting with a cyanating agent and, after developing 2-cyanopyrrolidines in the reaction system, it can be used without purification for step (b). This method is preferable for workers' safety, since 2-cyanopyrrolidines that may generate an extremely poisonous hydrocyanic acid gas can be reacted without purification.

While the solvent used for the extraction is not particularly limited, hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as methyl ethyl ketone, methyl isobutyl ketone and the like; carbonate solvents such as dimethyl carbonate, diethyl carbonate and the like; alcohol solvents such as methanol, ethanol, 2-propanol, 1-butanol, t-butanol and the like, and the like can be mentioned. Plural solvents selected therefrom may be used in a mixture at any ratio, or a reaction solvent alone may be used as an extraction solvent without adding a solvent during extraction. When the reaction solvent is water alone, extraction may be removal of the separated aqueous layer without using an organic solvent. As preferable extraction solvent, hexane, heptane, toluene, ethyl acetate, t-butanol, reaction solvent and a mixed solvent thereof can be mentioned. It is more preferable to perform extraction without addition of an organic solvent to increase producibility.

When a nitrogen atom on the pyrrolidine ring is protected, a protecting reagent and, where necessary, a base are used. Specific examples of the protection reagent include, but are not limited to, the following: acylating agents such as formic acid-acetic anhydride, acetic anhydride, acetyl chloride, chloroacetyl chloride, dichloroacetyl chloride, trichloroacetyl chloride, trifluoroacetic anhydride, propionyl chloride, benzoyl chloride, 4-chlorobenzoyl chloride and the like; alkoxycarbonylating agents such as methyl chloroformate, ethyl chloroformate, di-t-butyl dicarbonate, benzyl chloroformate (benzyloxycarbonyl chloride), allyl chloroformate (allyloxycarbonyl chloride) and the like; arylalkylating agents such as benzyl bromide, 4-methoxybenzyl bromide, 4-bromobenzyl bromide, 1-phenylethyl bromide and the like; and sulfonylating agents such as methanesulfonyl chloride, p-toluenesulfonyl chloride, 2-nitrobenzenesulfonyl chloride and the like.

Of these, preferred are acylating agent and alkoxycarbonylating agent that suppress decyanation decomposition from the 2-cyanopyrrolidine represented by the above-mentioned formula (7), more preferred are easily removable acetic anhydride, chloroacetyl chloride, trichloroacetyl chloride, trifluoroacetic anhydride, di-t-butyl dicarbonate, benzyloxycarbonyl chloride and allyloxycarbonyl chloride, particularly preferably industrially economical acetic anhydride and di-t-butyl dicarbonate.

Specific examples of the base to be used include, but are not limited to, the following: tertiary amines such as triethylamine, diisopropylethylamine, N-methylmorpholine, quinuclidine, 1,4-diazabicyclo[2.2.2]octane and the like; pyridines such as pyridine, 4-dimethylaminopyridine, 2,6-lutidine and the like; organic strong bases such as 1,8-diazabicyclo[5.4.0]undec-7-ene, tetramethylguanidine and the like; metal amides such as lithium diisopropylamide, sodium hexamethyldisilazide and the like; alkylmetals such as n-butyllithium, sec-butyllithium, tert-butyllithium, isopropylmagnesium bromide and the like; metal hydrides such as sodium hydride, calcium hydride and the like; metal alkoxides such as sodium methoxide, sodium ethoxide, potassium t-butoxide and the like; carbonates such as sodium hydrogen carbonate, potassium carbonate and the like; phosphates such as potassium phosphate, sodium hydrogen phosphate and the like; hydroxides such as sodium hydroxide, potassium hydroxide and the like; cyanides such as sodium cyanide, potassium cyanide and the like and the like.

Preferable base varies depending on the protecting reagent to be used. When acetic anhydride and di-t-butyl dicarbonate, which are preferable protecting reagents, are used, preferable base includes tertiary amines, pyridines, carbonates, hydroxides and cyanides, more preferably, cyanides showing an action to convert pyrroline obtained by decomposition of 2-cyanopyrrolidines to 2-cyanopyrrolidine again.

Step (b)

The α-substituted prolinamide represented by the above-mentioned formula (3) can be synthesized by hydrating the cyclic nitrogen-containing compound represented by the above-mentioned formula (2), preferably the 2-cyanopyrrolidine represented by the above-mentioned formula (7).

When the cyclic nitrogen-containing compound represented by the above-mentioned formula (2) is the pyrroline represented by the above-mentioned formula (6) free of a cyano group, it is preferably reacted with a cyanating agent to convert to the 2-cyanopyrrolidine represented by the above-mentioned formula (7) and then used for step (b). In this case, the 2-cyanopyrrolidine represented by the above-mentioned formula (7) may also be purified by a method such as extraction and the like, protected as necessary and then used for step (b). However, it is preferable to simplify the operation by allowing development of 2-cyanopyrrolidine in the reaction system, and then used for step (b) without purification. This method is preferable for workers' safety, since the 2-cyanopyrrolidine represented by the above-mentioned formula (7) that may generate an extremely poisonous hydrocyanic acid gas can be reacted without purification.

The hydration in step (b) can be performed in the presence of a catalyst that progresses the hydration reaction from nitrile to amide. Specific examples of the catalyst used in hydration include, but are not limited to, the following: hydrogen peroxide; hydroperoxides such as t-butyl hydroperoxide and the like; organic peracids such as peracetic acid, m-chloroperbenzoic acid and the like; inorganic peracids such as persulfuric acid, periodic acid and the like; inorganic acids such as hydrochloric acid, sulfuric acid, phosphoric acid and the like; organic acids such as trifluoromethanesulfonic

acid, methanesulfonic acid, trifluoroacetic acid and the like; inorganic bases such as sodium hydroxide, potassium carbonate and the like; complex catalysts such as [{Rh (OMe) (cod)}$_2$] $PCy_3$, {PtH($PMe_2OH$)($PMe_2O$)$_2$H} and the like; an enzyme having a nitrile hydratase activity and the like. Plural catalysts selected therefrom may be used in a mixture.

Of these, preferable catalyst varies depending on the substituent on the nitrogen of the 2-cyanopyrrolidine represented by the above-mentioned formula (7). When $R^2$ is a hydrogen atom, or an optionally substituted alkyl group, preferable catalysts are inorganic acid, organic acid, complex catalyst and enzyme that can suppress side reactions such as decomposition of unstable substrate and the like, more preferably, industrially economical inorganic acid.

On the other hand, when $R^2$ is an acetyl group or a t-butoxycarbonyl group, preferable catalysts are a combination of hydrogen peroxide water and inorganic base, complex catalyst and enzyme that can perform a hydration reaction under mild conditions, more preferably, a combination of industrially economical hydrogen peroxide water and inorganic base.

While a preferable amount of the catalyst to be used varies depending on the activity of the catalyst, it is generally 0.01 - 100 equivalents, preferably 0.02 - 20 equivalents, more preferably 0.1 - 10 equivalents, relative to the 2-cyanopyrrolidine represented by the above-mentioned formula (7).

In step (b), a solvent may be used where necessary. The solvent to be used is not particularly limited as long as it does not adversely influence the reaction. Specific examples thereof include hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as acetone, methyl ethyl ketone, methyl isobutyl ketone and the like; amide solvents such as dimethylformamide, N-methylpyrrolidone and the like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, t-butanol and the like; water and the like. Plural solvents selected therefrom may be used in a mixture at any ratio. Examples of preferable solvent include toluene, ethyl acetate, tetrahydrofuran, acetone, dimethylformamide, acetonitrile, dimethyl sulfoxide, alcohol solvents, water, and a mixed solvent thereof can be mentioned, more preferably alcohol solvents, water, and a mixed solvent thereof. As the amount of the solvent, any amount can be used. Generally, it is 0- to 50-fold volume, preferably 0- to 20-fold volume, more preferably 0- to 10-fold volume, relative to the 2-cyanopyrrolidine represented by the above-mentioned formula (7). Particularly, when the catalyst is a preferable inorganic acid, an excess solvent is not preferable since it decreases the activity of the catalyst. The preferable amount of the solvent to be used is 0- to 5-fold volume, more preferably 0- to 1-fold volume.

While the reaction temperature of step (b) is not particularly limited as long as the reaction is not adversely influenced, it is generally -20 - 120°C, preferably 10 - 80°C, more preferably 30 - 70°C.

While the reaction time of step (b) is not particularly limited as long as the reaction is not adversely influenced, it is preferably 10 min - 24 hr, more preferably 1 - 10 hr, to suppress the production cost.

The α-substituted prolinamide represented by the above-mentioned formula (3) obtained in step (b) can also be purified by a method such as extraction, distillation and/or crystallization and the like, or can also be used for the next step without purification. When $R^2$ is a hydrogen atom, a nitrogen atom on the pyrrolidine ring may be protected before use in step (c), and when $R^2$ is an amino-protecting group, the amino-protecting group may be removed before use in step (c).

While the solvent to be used for extraction and/or crystallization and the like is not particularly limited, hydrocarbon solvents such as hexane, heptane, cyclohexane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as methyl ethyl ketone, methyl isobutyl ketone and the like; carbonate solvents such as dimethyl carbonate, diethyl carbonate and the like; alcohol solvents such as 1-butanol, 2-butanol, 1-hexanol and the like, and the like can be mentioned. Plural solvents selected therefrom may be used in a mixture at any ratio. As preferable extraction solvent, hexane, heptane, cyclohexane, toluene, ethyl acetate, 1-butanol and a mixed solvent thereof can be mentioned.

Here, the crystallization includes, in addition to general crystallization wherein the object product is taken out as crystals by the addition of poor solvent, acid, base and the like to a solution, or decreasing the solubility by azeotropic distillation of good solvents such as water and the like, recrystallization comprising dissolving the once obtained crude crystal and the like in a suitable solvent and performing crystallization again. The crystal obtained here may be α-substituted prolinamides free of acid or base components, or a salt of α-substituted prolinamides with acid or base.

Step (c)

The optically active α-substituted proline represented by the above-mentioned formula (4) and/or the optically active α-substituted prolinamide represented by the above-mentioned formula (5) can be synthesized by resolving the

α-substituted prolinamide represented by the above-mentioned formula (3) by an enzymatical and/or chemical method. When the α-substituted prolinamide represented by the above-mentioned formula (3) does not have an asymmetric center other than the carbon atom to which a carbamoyl group is bonded, it can be synthesized by optical resolution of a racemate of the α-substituted prolinamide represented by the above-mentioned formula (3) or without sufficient optical purity. When plural asymmetric centers are present in a molecule, it can be synthesized by resolving a diastereomeric mixture of the α-substituted prolinamide represented by the above-mentioned formula (3) without a sufficient R:S ratio for the stereochemistry of the 2-position of pyrrolidine. While the method is not particularly limited as long as it can efficiently perform resolution, (d) asymmetric hydrolysis of amido group by an enzyme having an amidase activity; (e) resolution by diastereomeric salt formation; and (f) separation by column chromatography can be specifically mentioned. The resolution step in the present invention may be a single step of steps (d) to (f), or a combination of two or more from steps (d) to (f).

Step (d): asymmetric hydrolysis of amido group by an enzyme having an amidase activity

The enzyme having an amidase activity used in step (d) is not particularly limited as long as it is a substance derived from a living organism, which stereospecifically acts on racemic amino acid amide to convert same to amino acid. Examples of the form thereof include purified enzyme retaining an amidase activity (and immobilized product thereof), or a cell containing same, preparation of the cell (cell disruption, cell extract, crude purified enzyme, and immobilized product thereof), and culture medium obtained by culturing the cell. For example, an amidase produced by the bacteria or fungus shown below and commercially available enzyme are preferable.

Ochrobactrum anthropic NCIMB40321
Mycobacterium neoaurum ATCC25975
Rhizopus oryzae (e.g., enzyme for food additive, peptidase R (trade name) manufactured by Amano Enzyme Inc. etc.)

Of these, more preferred is a Rhizopus oryzae-derived enzyme (e.g., enzyme for food additive, peptidase R (trade name) manufactured by Amano Enzyme Inc. etc.) showing high selectivity in enzymatic resolution of α-methylprolinamide.

While the concentration of the enzyme having an amidase activity varies depending on the activity thereof, it is 0.0001- to 5-fold weight, preferably 0.001- to 1-fold weight, more preferably 0.001- to 0.1-fold weight, relative to the α-substituted prolinamide represented by the above-mentioned formula (3). An amount within this range is preferable in terms of reaction time, easy catalyst removal operation and the like.

In step (d), the reactivity can be improved by the addition of a compound that improves enzyme activity. While the additive to be used is not particularly limited, a divalent metal ion such as zinc, manganese, magnesium and the like, a reducing agent such as mercaptoethanol, dithiothreitol and the like, a non-ionic surfactant such as Triton X100 and the like, and a mixture thereof can be specifically mentioned. While the concentration of the compound that improves the enzyme activity varies depending on the activity thereof, it is generally preferably 0.0001 mass % - 1 mass %, relative to the amount of the reaction mixture. An amount within this range is preferable in terms of easy removal operation of the compound that improves the enzyme activity, the cost of the starting material and the like.

For step (d), the α-substituted prolinamide represented by the above-mentioned formula (3) having any substituent can be used. The substituent $R^2$ is preferably a hydrogen atom since solubility in water, which is a preferable solvent, becomes high.

Also, in step (d), α-substituted prolinamide having any purity can be used. Since enzyme reaction may be inhibited by impurity, a starting material purified by a method such as crystallization and the like is preferably used. Particularly, when substituent $R^2$ is a preferable hydrogen atom in step (d), α-substituted prolinamide shows basicity, and therefore, a salt with an acid is preferable since crystallinity is generally improved and the purification effect increases. Specific examples of the acid agent used here include, but are not limited to, the following: mineral acids such as hydrochloric acid, sulfuric acid, nitric acid and the like; carboxylic acids such as acetic acid, formic acid, trifluoroacetic acid, benzoic acid, oxalic acid, maleic acid, succinic acid and the like; and sulfonic acids such as methanesulfonic acid, toluenesulfonic acid and the like. Of these, preferred are mineral acid and sulfonic acids which are industrially economical and generally show high crystallinity of the salt, more preferably, hydrochloric acid, sulfuric acid, and toluenesulfonic acid, particularly preferably hydrochloric acid.

The solvent to be used in step (d) is not particularly limited as long as it does not adversely influence the reaction. Specific examples thereof include hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as acetone, methyl ethyl ketone, methyl isobutyl ketone and the like; amide solvents such as dimethylformamide, N-methylpyrrolidone and the

like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, t-butanol and the like; water and the like. Plural solvents selected therefrom may be used in a mixture at any ratio. Examples of preferable solvent include acetone, dimethylformamide, acetonitrile, dimethyl sulfoxide, alcohol solvents, water, and a mixed solvent thereof, and examples of more preferable solvent include single use of water at a low cost, and a mixed solvent of water and 10% by volume or below of acetone, dimethylformamide, acetonitrile, dimethyl sulfoxide or alcohol solvents relative to water.

As the amount of the solvent to be used, any amount of the solvent can be used. Generally, 1- to 200-fold volume relative to the α-substituted prolinamide represented by the above-mentioned formula (3) is preferable. An amount within this range is preferable in terms of producibility of the optically active amino acid. The amount of the solvent to be used is more preferably 1- to 50-fold volume, particularly preferably 1- to 10-fold volume, relative to the α-substituted prolinamide represented by the above-mentioned formula (3).

In step (d), adjustment of pH of the reaction mixture is preferable for preventing deactivation of an enzyme having an amidase activity and spontaneous decomposition of α-substituted prolinamides and affording the optimal catalytic activity and reaction yield. While an appropriate pH range varies depending on the enzyme having an amidase activity, it is preferably adjusted generally to a measurement value of 5.0 - 9.0, more preferably 6.0 - 8.0, at room temperature (specifically around 20 - 30°C). Since the α-substituted prolinamide represented by the above-mentioned formula (3) generally shows basicity in an aqueous solution, an acid is used for pH adjustment. In addition, when the α-substituted prolinamide represented by the above-mentioned formula (3) is used as a salt such as hydrochloride and the like, since it generally shows neutral acidity to weak acidity in an aqueous solution, an acid or base is used for pH adjustment. Alternatively, an inorganic acid, which is a preferable catalyst, is used in step (b) and, since an acidic aqueous solution of the obtained α-substituted prolinamide represented by the above-mentioned formula (3) generally shows acidity when directly used for the reaction, a base is used for pH adjustment.

When the pH of the solution changes during reaction, an acid or base may be appropriately added to adjust the pH. In this case, pH is preferably adjusted to a measurement value of 5.0 - 9.0, more preferably 6.0 - 8.0, at 20 - 30°C.

Specific examples of the acid or base to be used include the following, and are not particularly limited as long as it can adjust to an appropriate pH: mineral acids such as hydrochloric acid, sulfuric acid, phosphoric acid and the like; organic acids such as methanesulfonic acid, trifluoroacetic acid, acetic acid, formic acid and the like; carbonates such as sodium hydrogen carbonate, potassium carbonate and the like; phosphates such as potassium phosphate, sodium hydrogen phosphate and the like; hydroxides such as sodium hydroxide, potassium hydroxide and the like, and the like. Of these, an economical mineral acid is preferably used as an acid, and an economical hydroxide is preferably used as a base. As a use form, they can be used as a compound per se or an aqueous solution thereof.

While the reaction temperature of step (d) is not particularly limited as long as the reaction is not adversely influenced, it is preferably within the range of 5 - 70°C. A temperature within the range is preferable in terms of reaction time, reaction yield and the like, 15 - 60°C is more preferable, and 25 - 50°C is particularly preferable.

While the reaction time of step (d) is not particularly limited as long as the reaction is not adversely influenced, it varies depending on the amount of the catalyst, and the kind of the α-substituted prolinamide represented by the above-mentioned formula (3) and is generally 5 - 60 hr. A time within the range is preferable in terms of reaction yield, operational efficiency of production step and the like. The termination of the reaction of steroselective hydrolysis by an enzyme having an amidase activity is preferably at not less than 90%, more preferably not less than 98%, of the theoretical value of the yield and/or conversion ratio as calculated from the quantified value of α-substituted prolines by high performance liquid chromatography. The diastereomeric purity and/or optical purity are calculated from the area ratio and/or quantitative value of α-substituted prolines and/or α-substituted prolinamides by high performance liquid chromatography and, when the asymmetric center is present only at the carbon atom to which a carbamoyl group is bonded, the termination of the reaction at not less than 80%ee is preferable, not less than 95%ee is more preferable. Since pharmaceutical products and intermediates therefor require high optical purity, not less than 99%ee is particularly preferable.

When plural asymmetric centers are present in a molecule, the termination of the reaction is preferably at R form:S form (molar ratio)=90:10 - 100:0 or R form:S form (molar ratio)=10:90 - 0:100 for the stereochemistry of the 2-position of pyrrolidine, more preferably at R form:S form (molar ratio)=97.5:2.5 - 100:0 or R form:S form (molar ratio)=2.5:97.5

- 0:100. Since pharmaceutical products and intermediates therefor require high optical purity, termination of the reaction is more preferably at R form:S form (molar ratio)=99:1
- 100:0 or R form:S form (molar ratio)=1:99 - 0:100, particularly preferably R form:S form (molar ratio)=99.5:0.5-100:0 or R form:S form (molar ratio)=0.5:99.5 - 0:100.

As for the termination of the reaction in step (d), discontinuation of the activity of an enzyme having an amidase

activity is preferable for suppressing a decrease in the optical purity and yield due to the excessive progress of the reaction. Specific examples of the operation include a method including deactivation of the enzyme having an amidase activity by adjustment of pH and temperature, a method including casting an additive for inhibiting the activity of an enzyme having an amidase activity, or removing by coagulation of the activity of an enzyme having an amidase activity and the like.

The optically active α-substituted proline represented by the above-mentioned formula (4) and/or the optically active α-substituted prolinamide represented by the above-mentioned formula (5) obtained in step (d) can be purified by methods such as filtration, extraction, distillation, separation by resin and/or crystallization and the like. When $R^2$ is a hydrogen atom, the nitrogen atom on the pyrrolidine ring may be protected after step (d) and when $R^2$ is an amino-protecting group, the amino-protecting group may be removed after step (d).

Filtration includes, in addition to filtration using a filter such as ultrafiltration membrane, microfiltration membrane, filter cloth, filter paper and the like, filtration using adsorbent such as celite, activated carbon and the like. It is also possible to facilitate removal of the components derived from living organisms by adding, to the reaction mixture, an inorganic coagulating agent such as aluminum oxide, magnesium chloride, calcium chloride and the like, or a polymer coagulating agent such as polyethyleneimine, chitosan and the like, singly or in mixture. In addition, an enzyme having an amidase activity contained in a fraction concentrated by filtration can also be reused.

While the solvent used for extraction and/or crystallization and the like is not particularly limited, hydrocarbon solvents such as hexane, heptane, cyclohexane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, butyl acetate and the like; ketone solvents such as acetone, methyl ethyl ketone, methyl isobutyl ketone and the like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, 1-butanol, 2-butanol, t-butanol and the like; water and the like can be mentioned. Plural solvents selected therefrom may be used in a mixture at any ratio.

As preferable extraction solvents, toluene, ethyl acetate, tetrahydrofuran, methyl ethyl ketone, 1-butanol, 2-butanol, t-butanol and a mixed solvent thereof can be mentioned. In addition, it is preferable to maintain basicity of the aqueous layer, since the optically active α-substituted proline represented by the above-mentioned formula (4) can be partitioned in an aqueous layer, and the optically active α-substituted prolinamide represented by the above-mentioned formula (5) can be partitioned in an organic layer, and addition of sodium chloride and the like is preferable for enhancing the extraction efficiency by increasing the salt concentration of the aqueous layer.

As preferable crystallization solvents, toluene, ethyl acetate, acetone, methanol, ethanol, t-butanol, 2-butanol, 1-butanol, water, a mixed solvent thereof can be mentioned. Since the optically active α-substituted proline represented by the above-mentioned formula (4) and the optically active α-substituted prolinamide represented by the above-mentioned formula (5) generally have markedly different solubilities in solvent, separation by crystallization utilizing the difference in solubility is preferable. In the preferable embodiment of the optically active α-substituted proline represented by the above-mentioned formula (4) wherein $R^2$ is a hydrogen atom, since the amino acid has both a carboxyl group and an amino group, crystallization by adjusting the pH of the solution to the isoelectric point and decreasing the solubility is preferable, and crystallization by decreasing the solubility by adding a salt such as sodium chloride and the like or an organic solvent is further preferable.

The resin used for separation by resin is not particularly limited as long as it has an ability to separate the optically active α-substituted proline represented by the above-mentioned formula (4), the optically active α-substituted prolinamide represented by the above-mentioned formula (5) and/or other impurity. Specific examples thereof include strongly acidic cation exchange resin, strongly basic anion exchange resin, weakly acidic cation exchange resin, and weakly basic anion exchange resin. They may be used in combination to afford a sufficient degree of purification. Of these, preferred are strongly basic anion exchange resin and weakly basic anion exchange resin, which can separate them by adsorbing the optically active α-substituted proline represented by the above-mentioned formula (4) on the resin and not adsorbing the optically active α-substituted prolinamide represented by the above-mentioned formula (5) on the resin, and the more preferred is a strongly basic anion exchange resin having a strong adsorption power.

While the purification step is not particularly limited as to its method as long as the necessary degree of purification can be obtained, to increase the productivity, it is preferable to use industrially less burdensome methods in combination. Specifically, a method including ultrafiltration of the reaction mixture, applying the filtrate onto a strongly basic anion exchange resin to separate the optically active α-substituted proline represented by the above-mentioned formula (4) and the optically active α-substituted prolinamide represented by the above-mentioned formula (5), and crystallizing the obtained aqueous solution to give the highly pure optically active α-substituted proline represented by the above-mentioned formula (4) and/or optically active α-substituted prolinamide represented by the above-mentioned formula (5) can be mentioned.

Step (e): resolution by diastereomeric salt formation

The resolution by diastereomeric salt formation in step (e) is a resolution method including reacting an optically active acid when the α-substituted prolinamide represented by the above-mentioned formula (3) is a racemate, and an optically active acid or achiral acid when α-substituted prolinamide is a diastereomeric mixture having plural asymmetric centers, and separating the resultant crystalline salt by filtration and the like. By this operation, the optically active α-substituted prolinamide represented by the above-mentioned formula (5) is obtained in the crystal and/or filtrate.

Specific examples of the optically active acid used in step (e) include, but are not limited to, the following: tartaric acids such as L-tartaric acid, D-tartaric acid, (2S,3S)-dibenzoyltartaric acid, (2R,3R)-dibenzoyltartaric acid, (2S,3S)-di(p-toluoyl)tartaric acid, (2R,3R)-di(p-toluoyl)tartaric acid and the like; mandelic acids such as (S)-mandelic acid, (R)-mandelic acid and the like; amino acid derivatives such as N-acetyl-L-alanine, N-acetyl-L-phenylglycine, N-acetyl-D-phenylglycine, N-benzyl-L-phenylglycine, N-benzyl-D-phenylglycine, N-acetyl-L-phenylalanine, N-acetyl-L-glutamic acid, N-acetyl-L-aspartic acid and the like; optically active sulfonic acids such as (S)-10-camphorsulfonic acid, (R)-10-camphorsulfonic acid, (S)-1-phenylethanesulfonic acid, (R)-1-phenylethanesulfonic acid and the like, and the like. Of these, preferred are industrially economical tartaric acids or mandelic acids generally showing high resolution ability.

Specific examples of the achiral acid used in step (e) include, but are not limited to, the following: mineral acids such as hydrochloric acid, sulfuric acid, nitric acid and the like; carboxylic acids such as acetic acid, formic acid, trifluoroacetic acid, benzoic acid, oxalic acid, maleic acid, succinic acid and the like; and sulfonic acids such as methanesulfonic acid, toluenesulfonic acid and the like. Of these, preferred are industrially economical hydrochloric acid, acetic acid, benzoic acid, oxalic acid, maleic acid, succinic acid, toluenesulfonic acid generally showing high crystallinity.

When the optically active acid and/or achiral acid to be used is/are not less than divalent acid having plural acidic groups, the resulting diastereomeric salt may be a 1:1 salt or 1:2 or more salt.

The amount of the optically active acid and/or achiral acid to be used is 0.1 - 10 equivalents, since excessive use prevents crystallization, preferably 0.2 - 3 equivalents, more preferably 0.3 - 1 equivalent, relative to the α-substituted prolinamide represented by the above-mentioned formula (3). The amount of use here means the equivalent amount of acid used for salt formation.

While the solvent to be used in step (e) is not particularly limited, hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, isopropyl acetate, butyl acetate and the like; ketone solvents such as acetone, methyl ethyl ketone, methyl isobutyl ketone and the like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, 1-butanol, 2-butanol, t-butanol and the like; water and the like can be mentioned. Plural solvents selected therefrom may be used in a mixture at any ratio.

A preferable solvent is one showing sufficient dissolution of the α-substituted prolinamide represented by the above-mentioned formula (3), optically active acid, or achiral acid, and sufficient low solubility of the resulting diastereomeric salt, and examples thereof include ether solvents such as ethyl ether, propyl ether, cyclopentyl methyl ether, t-butyl methyl ether, tetrahydrofuran and the like; ester solvents such as ethyl acetate, isopropyl acetate, butyl acetate and the like; alcohol solvents such as methanol, ethanol, 2-propanol, 1-butanol, 2-butanol, t-butanol and the like, and a mixed solvent of these solvents and any solvent, and more preferred are ester solvents such as ethyl acetate, isopropyl acetate, butyl acetate and the like, and a mixed solvent of these solvents and any solvent.

Moreover, crystallization may be promoted by adding a solvent showing low solubility of diastereomeric salt to the reaction solution. As the solvent in this case, hydrocarbon solvents such as hexane, heptane, benzene, toluene and the like; ester solvents such as ethyl acetate, isopropyl acetate, butyl acetate and the like can be mentioned.

The amount of the solvent to be used is, generally 1- to 50-fold volume relative to the α-substituted prolinamide represented by the above-mentioned formula (3), since an amount sufficient to ensure flowability of the precipitated diastereomeric salt is necessary and a smaller amount of use leads to higher producibility, preferably 2- to 20-fold volume, more preferably 3- to 10-fold volume.

A seed crystal may be added to induce crystallization. The diastereomeric salt obtained in step (e) can be used as seed crystals. Alternatively, it may be obtained by dissolving the optically active α-substituted prolinamide represented by the above-mentioned formula (5) having high purity and optically active acid, or achiral acid, followed by an operation such as concentration to dryness, cooling, physical impact and the like.

While the temperature in step (e) is not particularly limited, it is generally -20 - 120°C, preferably -10 - 80°C, more preferably 0 - 70°C. When seed crystals are used, it is preferable to add the seed crystals at a high temperature at which the solubility is comparatively high, and gradually cool the mixture, so as to obtain crystals with high purity.

In step (e), resolution may be achieved by increasing the diastereomeric purity and/or optical purity of the filtrate. Since it is generally easy to increase the purity of crystal, the resolution is preferably achieved by increasing the

diastereomeric purity of the diastereomeric salt obtained as crystals. When the diastereomeric purity and/or optical purity of the obtained optically active α-substituted prolinamide represented by the above-mentioned formula (5) are/is insufficient, the purity is preferably increased by a method such as recrystallization and the like.

When the diastereomeric purity and/or optical purity of the optically active α-substituted prolinamide represented by the above-mentioned formula (5) obtained in step (e) may be any values. When the purity is low, it needs to be increased outside this step. Therefore, the stereochemistry of the 2-position of pyrrolidine is preferably R form:S form (molar ratio)=90:10 - 100:0 or R form:S form (molar ratio)=10:90-0:100, more preferably R form:S form (molar ratio)=97.5:2.5-100:0 or R form:S form (molar ratio)=2.5:97.5 - 0:100. Since pharmaceutical products and intermediates therefor require high optical purity, it is more preferably R form:S form (molar ratio)=99:1 - 100:0 or R form:S form (molar ratio)=1:99 - 0:100, particularly preferably R form:S form (molar ratio)=99.5:0.5-100:0 or R form:S form (molar ratio)=0.5:99.5 - 0:100.

The diastereomeric salt of the optically active α-substituted prolinamide represented by the above-mentioned formula (5) obtained in step (e) can be separated into an optically active α-substituted prolinamide and an optically active acid and/or achiral acid by a method such as extraction and the like. The separated optically active acid and/or achiral acid may be recovered and reused.

When $R^2$ in the optically active α-substituted prolinamide represented by the above-mentioned formula (5) is a hydrogen atom, the nitrogen atom on the pyrrolidine ring may be protected after step (e) and when $R^2$ is an amino-protecting group, the amino-protecting group may be removed after step (e). In addition, the optically active α-substituted prolinamide represented by the above-mentioned formula (5) may be hydrolyzed for conversion to the optically active α-substituted proline represented by the above-mentioned formula (4). When the reaction is performed successively after step (e), the above-mentioned diastereomeric salt may be used as a starting material, or the optically active α-substituted prolinamide represented by the above-mentioned formula (5) after separation of the optically active acid and/or achiral acid may be used as a starting material.

The conversion of the optically active α-substituted prolinamide represented by the above-mentioned formula (5) to the optically active α-substituted proline represented by the above-mentioned formula (4) by hydrolysis can be performed by any known method using a hydrolysis catalyst in the presence of 1 equivalent or more of water.

Specific examples of the hydrolysis catalyst to be used include, but are not limited to, the following: inorganic acids such as hydrochloric acid, sulfuric acid, phosphoric acid and the like; organic acids such as trifluoromethanesulfonic acid, methanesulfonic acid, trifluoroacetic acid and the like; inorganic bases such as sodium hydroxide, potassium carbonate and the like; enzymes having amidase or peptidase activity and the like. Plural catalysts selected therefrom may be used in a mixture.

Step (f): separation by column chromatography

The separation by column chromatography in step (f) is a method of separating each diastereomer by passing the α-substituted prolinamide represented by the above-mentioned formula (3) which is a diastereomeric mixture through a column packed with an achiral filler. By this operation, the optically active α-substituted prolinamide represented by the above-mentioned formula (5) can be obtained.

Examples of the column chromatography used in step (f) include, but are not limited to, the following: silica gel column chromatography using spherical silica gel (neutral), spherical silica gel (acidic); reversed-phase column chromatography using silica gel wherein straight chain alkyl group having 18 carbon atoms, 8 carbon atoms and the like is bonded; column chromatography using styrene-divinylbenzene-based synthetic adsorbent and the like. Specific examples of the synthetic adsorbent include HP20, HP21, SP70, SP207, SP700, SP825, SP850 manufactured by Mitsubishi Chemical Corporation and the like. Of these, preferred is economical and repeatedly utilizable silica gel column chromatography and column chromatography using a synthetic adsorbent, more preferably column chromatography using a synthetic adsorbent and permitting use of an economical aqueous solvent as an eluent.

The method of separation may be batch type column chromatography wherein a charged sample is completely eluted every time, or continuous column chromatography using a simulated moving bed.

The solvent to be used in step (f) is not particularly limited, hydrocarbon solvents such as hexane, heptane, benzene, toluene and the like; ether solvents such as ethyl ether, propyl ether, cyclopentyl methyl ether, t-butyl methyl ether, tetrahydrofuran and the like; halogenated hydrocarbon solvents such as dichloromethane, chloroform, dichloroethane, chlorobenzene and the like; ester solvents such as ethyl acetate, isopropyl acetate, butyl acetate and the like; ketone solvents such as acetone, methyl ethyl ketone, methyl isobutyl ketone and the like; carbonate solvents such as dimethyl carbonate, diethyl carbonate and the like; nitrile solvents such as acetonitrile and the like; sulfur-containing solvents such as dimethyl sulfoxide, sulfolane and the like; alcohol solvents such as methanol, ethanol, 2-propanol, 1-butanol, 2-butanol, t-butanol and the like; water and the like. Plural solvents selected therefrom may be used in a mixture at any ratio.

While a preferable solvent varies depending on the kind of column chromatography to be used, when column

chromatography using a preferable synthetic adsorbent is used, ketone solvents; nitrile solvents; alcohol solvents; and water are preferable, and economical acetone, methanol, and water are more preferable.

Moreover, an additive for pH control may also be added where necessary. Specific examples of the additive include acids such as acetic acid, formic acid and the like; salts such as ammonium acetate, sodium acetate, ammonium chloride and the like; bases such as ammonia, sodium hydroxide and the like, and the like. These may be used in a mixture at any mixing ratio.

When the optically active α-substituted prolinamide represented by the above-mentioned formula (5) obtained in step (f) has an asymmetric center only at the carbon atom to which a carbamoyl group is bonded, not less than 80%ee is preferable, more preferably 90%ee, not less than 95%ee is more preferable. Since pharmaceutical products and intermediates therefor require high optical purity, not less than 99%ee is particularly preferable. When plural asymmetric centers are present in a molecule, the stereochemistry of the 2-position of pyrrolidine is preferably R form:S form (molar ratio)=90:10-100:0 or R form:S form (molar ratio)=10:90 - 0:100, more preferably R form:S form (molar ratio)=97.5:2.5 - 100:0 or R form:S form (molar ratio)=2.5:97.5 - 0:100. Since pharmaceutical products and intermediates therefor require high optical purity, it is more preferably R form:S form (molar ratio)=99:1 - 100:0 or R form:S form (molar ratio)=1:99.5-0:100, particularly preferably R form:S form (molar ratio)=99.5:0.5 - 100:0 or R form:S form (molar ratio)=0.5:99.5 - 0:100.

Examples

The present invention is explained in more detail in the following by referring to Examples, which are not to be construed as limitative.

[Example 1]

Production of 2-cyano-2-methylpyrrolidine and 2-methyl-1-pyrroline (in the above-mentioned formula (2), $R^1$=Me, $R^2$=$R^3$=H; step (a) using sodium cyanide and ammonium acetate, reaction in methanol-water solvent)

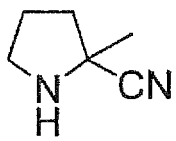

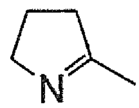

In a flask were charged 5-chloro-2-pentanone (2.41 g, 20 mmol), sodium cyanide (1.08 g, 22 mmol), ammonium acetate (4.62 g, 60 mmol), water (5 ml) and methanol (2.5 ml), and the mixture was reacted at 50°C for 3 hr. To the reaction mixture was added ethyl acetate and 50 w/v% NaOH aqueous solution (3.2 ml) for partitioning, and the aqueous layer was reextracted with ethyl acetate. The organic layer was washed with saturated brine, and the solvent was evaporated at 35°C, 80 hPa to give a brown oily substance (2.31 g). From the results of NMR analysis, this oily substance was a mixture containing 2-cyano-2-methylpyrrolidine (60 wt%, 12.5 mmol, yield 62%), 2-methyl-1-pyrroline (7 wt%, 2.0 mmol, yield 10%) and ethyl acetate (34 wt%).
2-cyano-2-methylpyrrolidine: $^1$H-NMR(400MHz,$CDCl_3$) δ 1.58(3H,s), 1.72 (1H, ddd, J=12.6, 9.6, 8.3Hz), 1.83-2.08 (2H,m), 2.26 (1H, ddd, J=12.6, 8.3, 4.3Hz), 3.12-3.22 (2H,m).

[Example 2]

Production of α-methylprolinamide hydrochloride (in the above-mentioned formula (3), $R^1$=Me, $R^2$=$R^3$=H; step (b) hydration with hydrochloric acid)

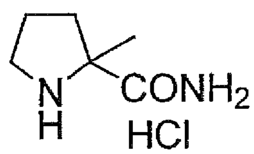

2-Cyano-2-methylpyrrolidine (0.40 g, 60 wt%, 2.2 mmol) obtained in Example 1 and concentrated hydrochloric acid (1 ml) were charged in a flask, and the mixture was reacted at room temperature for 15 hr, and at 50°C for 5 hr. After cooling to room temperature, 50 w/v% NaOH aqueous solution (1 ml) was added, and the mixture was extracted 6 times with ethyl acetate. The organic layer was dried over magnesium sulfate and concentrated to give crude 2-methylprolinamide. To the crude product were added methanol (0.1 ml), ethyl acetate (0.25 ml) and 4 N hydrochloric acid-ethyl acetate solution (0.5 ml), and the mixture was ice-cooled. The crystals were collected by filtration to give α-methylprolinamide hydrochloride (92 mg, 0.56 mmol, yield 26%) as pale-brown crystals.
$^1$H-NMR (400MHz, $CD_3OD$) δ 1.69 (3H,s), 1.96-2.22(3H,m), 2.36-2.44(1H,m), 3.34-3.44 (2H,m) .

[Example 3]

Production of (S)-α-methylproline and (R)-α-methylprolinamide (in the above-mentioned formulas (4) and (5), $R^1$=Me, $R^2$=$R^3$=H; step (d) enzymatic resolution of racemate)

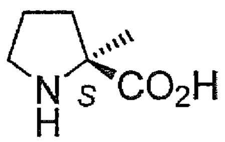 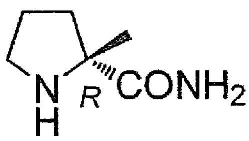

In a 2 ml sample tube was prepared α-methylprolinamide hydrochloride obtained according to the method of Example 2 to a final concentration of 500 g/L with 0.2 M Tris buffer (pH 7.0) and the mixture was adjusted to pH 6.8 with 3% aqueous sodium hydroxide. To this mixture (0.8 mL) was added peptidase R (trade name, manufactured by Amano Enzyme Inc., derived from Rhizopus oryzae) aqueous solution (0.2 mL) prepared to 50 g/L, and the mixture was reacted at 40, stirring number 800 rpm for 161 hr. As a result of purity analysis and optical purity analysis by HPLC analysis, (S)-α-methylproline had a pure content of 0.153 mg (1.18 mmol, yield 48.6%) and an optical purity of 99.3%ee, and the E value of enzyme in this reaction was 1024.
The conditions of optical purity analysis by HPLC were as described below.
column: CLC-D (4.6 mm × 150 mm) manufactured by ASTEC, mobile
phase: 2 mM $CuSO_4$ aqueous solution, flow rate: 1.0 mL/min,
column temperature: 45, UV: 254 nm
The E value was calculated by the following formula from the conversion ratio (c) of the reaction and the optical purity (eeS) of the residual substrate.

$$E=\ln[(1-c)(1-eeS)]/\ln[(1-c)(1+eeS)]$$

[Example 4]

Production of 2-cyano-2-methylpyrrolidine and 2-methyl-1-pyrroline (in the above-mentioned formula (2), $R^1$=Me, $R^2$=$R^3$=H; step (a) reaction in methanol solvent)

5-Chloro-2-pentanone (0.228 ml, 2.0 mmol), sodium cyanide (108 mg, 2.2 mmol), ammonium acetate (462 mg, 6.0 mmol) and methanol (1 ml) were charged in a flask, and the mixture was reacted at 50°C for 1 hr. The reaction mixture was analyzed based on NMR. As a result, 5-chloro-2-pentanone disappeared, and 2-cyano-2-methylpyrrolidine and 2-methyl-1-pyrroline were produced at a 1:0.4 ratio.

[Example 5]

Production of 2-cyano-2-methylpyrrolidine and 2-methyl-1-pyrroline (in the above-mentioned formula (2), $R^1$=Me, $R^2$=$R^3$=H; step (a) reaction in t-butanol-water solvent)

5-Chloro-2-pentanone (4.80 g, 40 mmol), sodium cyanide (2.35 g, 48 mmol), ammonium acetate (6.2 g, 80 mmol), water (10 ml) and t-butanol (20 ml) were charged in a flask, and the mixture was reacted at 50°C for 2.5 hr. Sodium cyanide (1.25 g, 26 mmol) was added, and the mixture was further reacted for 2.5 hr. To the reaction mixture

were added ethyl acetate and 50 w/v% NaOH aqueous solution (2.1 g) for partitioning, and the aqueous layer was reextracted with ethyl acetate. The organic layer was dried over sodium sulfate, and the solvent was evaporated to give a yellow oily substance (4.2 g). From the results of NMR analysis, this oily substance was a mixture containing 2-cyano-2-methylpyrrolidine (67 wt%, 2.8 g, yield 64%), 2-methyl-1-pyrroline (14 wt%, 0.59 g, yield 18%), ethyl acetate (10 wt%) and t-butanol (9 wt%).

[Example 6]

Production of α-methylprolinamide hydrochloride (in the above-mentioned formula (3), $R^1$=Me, $R^2$=$R^3$=H; step (a) reaction in isopropyl acetate-water solvent, step (b) hydration with sulfuric acid)

5-Chloro-2-pentanone (70 g, 0.58 mol), sodium cyanide (34.1 g, 0.70 mmol), ammonium acetate (134 g, 1.74 mol), water (168 ml) and isopropyl acetate (280 ml) were charged in a flask, and the mixture was reacted at 60°C for 3 hr. Sodium cyanide (17.1 g, 0.35 mol) was added, and the mixture was further reacted for 6 hr. After cooling to room temperature, the aqueous layer was removed, the organic layer was filtered through celite, and washed with isopropyl acetate (20 ml) to give a solution of 2-cyano-2-methylpyrrolidine in isopropyl acetate.

Water (25 ml) and sulfuric acid (56.9 g, 0.58 mol) were charged in another flask, and the solution of 2-cyano-2-methylpyrrolidine in isopropyl acetate was added dropwise under ice-cooling. After standing, the isopropyl acetate layer was removed, and the sulfuric acid layer was washed with isopropyl acetate (35 ml). Furthermore, sulfuric acid (114 g, 1.39 mol) was added, and the mixture was reacted at 25 - 30°C for 1 day. The reaction mixture was ice-cooled, water (140 ml), 40 wt% aqueous sodium hydroxide solution (377 g) and 1-butanol (210 ml) were slowly added. The resulting crystals of sodium sulfate were filtered off, washed with 1-butanol (200 ml) and methanol (300 ml) and the filtrate was concentrated to 290 g. The aqueous layer was removed to give a solution of α-methylprolinamide in 1-butanol. To this solution were added concentrated hydrochloric acid (41 g, 0.39 mol) and cyclohexane (28 ml), and the mixture was subjected to azeotropic dehydration under normal pressure with heating under reflux by Dean-Stark apparatus. After cooling to room temperature, the crystals were collected by filtration, washed with 1-butanol and ethyl acetate and dried under reduced pressure to give α-methylprolinamide hydrochloride (46.8 g) as pale-brown crystals. purity 100 wt% (HPLC analysis), 0.284 mol, yield 49%.

[Example 7]

Production of α-methylprolinamide hydrochloride (in the above-mentioned formula (3), $R^1$=Me, $R^2$=$R^3$=H; step (a) reaction in ethyl acetate-water solvent, step (b) hydration with sulfuric acid)

5-Chloro-2-pentanone (70 g, 0.58 mol), sodium cyanide (17.1 g, 0.35 mmol), ammonium acetate (134 g, 1.74 mol), water (168 ml) and ethyl acetate (280 ml) were charged in a flask, and the mixture was heated to 60°C. Sodium cyanide (17.1 g, 0.35 mol) was added twice 1.5 hr and 3.5 hr later, and the mixture was further reacted for 5 hr. After cooling to room temperature, the aqueous layer was removed to give a solution of 2-cyano-2-methylpyrrolidine in ethyl acetate.

In another flask were charged water (10 ml) and sulfuric acid (56.9 g, 0.58 mol) and the solution of 2-cyano-2-methylpyrrolidine in ethyl acetate was added dropwise under ice-cooling. After standing, the ethyl acetate layer was removed, and the sulfuric acid layer was washed with ethyl acetate (35 ml). Furthermore, sulfuric acid (114 g, 1.39 mol) was added, and the mixture was reacted at 20 - 40°C for 6 hr. The reaction mixture was ice-cooled, water (140 ml), 40 wt% aqueous sodium hydroxide solution (384 g) and methanol (210 ml) were slowly added. The resulting crystals of sodium sulfate were filtered off and washed with methanol (350 ml), and the filtrate was concentrated to 300 g. 1-Butanol (280 ml) was added, the aqueous layer was removed, and the aqueous layer was reextracted with 1-butanol (70 ml) to give a solution of α-methylprolinamide in 1-butanol. To this solution were added concentrated hydrochloric acid (49 g, 0.47 mol) and cyclohexane (70 ml), and the mixture was subjected to azeotropic dehydration under normal pressure with heating under reflux by Dean-Stark apparatus. After cooling to room temperature, the crystals were collected by filtration, washed with 1-butanol and ethyl acetate and dried under reduced pressure to give α-methylprolinamide hydrochloride (65.6 g) as pale-yellow crystals. purity 95 wt% (HPLC analysis), 0.380 mol, yield 65%.

[Example 8]

Production of α-methylprolinamide hydrochloride (in the above-mentioned formula (3), $R^1$=Me, $R^2$=$R^3$=H; step (a) reaction in ethyl acetate-water solvent, step (b) hydration with sulfuric acid)

Ammonium acetate (95.9 g, 1.25 mol), water (100 mL), ethyl acetate (200 mL), 5-chloro-2-pentanone (50 g,

0.42 mol) and sodium cyanide (12.2 g, 0.25 mol) were charged in a flask, and the mixture was heated to 60°C. 28% Aqueous ammonia (28.8 g) was added by portions, and the mixture was reacted while adjusting pH to 7.4-7.6. After 3 hr, sodium cyanide (12.2 g, 0.25 mol) was added, and the mixture was further reacted for 3 hr. After cooling to room temperature, the aqueous layer was removed to give a solution of 2-cyano-2-methylpyrrolidine in ethyl acetate.

In a different flask was charged sulfuric acid (40.7 g, 0.42 mol), and the solution of 2-cyano-2-methylpyrrolidine in ethyl acetate was added dropwise under ice-cooling. After standing, the ethyl acetate layer was removed, and the sulfuric acid layer was washed twice with cyclohexane (50 mL). Sulfuric acid (81.4 g, 0.83 mol) was further added, and the mixture was reacted at 40 - 60°C for 4 hr. The reaction mixture was ice-cooled, and water (180 mL) and 28% aqueous ammonia (151 g) were slowly added. To the obtained solution was added 1-butanol (330 mL), the aqueous layer was removed, and the aqueous layer was reextracted with 1-butanol (165 mL) to give a solution of α-methylprolinamide in 1-butanol. This solution was concentrated to remove water, and the precipitated solid content was filtered off. Concentrated hydrochloric acid (27.3 g, 0.26 mol) and cyclohexane (50 mL) were added, and the mixture was subjected to azeotropic dehydration under normal pressure with heating under reflux by Dean-Stark apparatus. After cooling to room temperature, the crystals were collected by filtration, washed with 1-butanol and ethyl acetate and dried under reduced pressure to give α-methylprolinamide hydrochloride (29.7 g) as pale-yellow crystals. purity 94 wt% (HPLC analysis), 0.171 mol, yield 41%.

[Example 9]

Production of 2-cyano-2-methylpyrrolidine and 2-methyl-1-pyrroline (in the above-mentioned formula (2), $R^1$=Me, $R^2$=$R^3$=H; step (a) using acetic acid and aqueous ammonia, reaction in water solvent)

5-Chloro-2-pentanone (0.57 ml, 5.0 mmol), sodium cyanide (270 mg, 5.5 mmol), acetic acid (0.32 ml, 5.5 mmol), 28% aqueous ammonia (0.61 ml, 10 mmol) and water (0.57 ml) were charged in a flask, and the mixture was reacted at 40°C for 6 hr. 50 w/v% NaOH aqueous solution (0.4 ml) was added, and the mixture was further reacted at 60°C for 1 hr. The reaction mixture was cooled to room temperature, and the organic layer and the aqueous layer were separated. The aqueous layer was extracted with ethyl acetate, and the combined organic layer was quantified by NMR using toluene as an internal standard. As a result, it contained 2-cyano-2-methylpyrrolidine (2.27 mmol, yield 45%) and 2-methyl-1-pyrroline (0.59 mmol, yield 12%).

[Example 10]

Production of 2-cyano-2-methylpyrrolidine and 2-methyl-1-pyrroline (in the above-mentioned formula (2), $R^1$=Me, $R^2$=$R^3$=H; step (a) using ammonium chloride, reaction in water solvent)

5-Chloro-2-pentanone (1.14 ml, 10 mmol), sodium cyanide (540 mg, 11 mmol), ammonium chloride (1.07 g, 20 mmol) and water (2.3 ml) were charged in a flask, and the mixture was reacted at 40°C for 6 hr. 28% Aqueous ammonia (0.61 ml, 10 mmol) was added, and the mixture was further reacted at 60°C for 1 hr. The reaction mixture was cooled to room temperature, 50 w/v% NaOH aqueous solution (0.4 ml) was added, and the mixture was extracted with ethyl acetate. The organic layer was quantified by NMR using toluene as an internal standard. As a result, it contained 2-cyano-2-methylpyrrolidine (5.7 mmol, yield 57%) and 2-methyl-1-pyrroline (0.60 mmol, yield 6%).

[Example 11]

Production of α-methylprolinamide hydrochloride (in the above-mentioned formula (3), $R^1$=Me, $R^2$=$R^3$=H, step (a) using ammonium acetate, reaction in water solvent; step (b) hydration with sulfuric acid)

Ammonium acetate (38.4 g, 0.50 mol), water (30 mL), sodium cyanide (9.8 g, 0.20 mol) and 5-chloro-2-pentanone (20.0 g, 0.17 mol) were charged in a flask, and the mixture was reacted at 50°C for 30 min. 28% Aqueous ammonia (12.0 g) was added, and the mixture was reacted for 2 hr, and cooled to room temperature, and the aqueous layer was removed. The organic layer in the flask was washed with cyclohexane (10 mL), concentrated sulfuric acid (48.8 g, 0.50 mol) was added dropwise to the obtained organic layer under ice-cooling, and the mixture was reacted at room temperature for 4 hr. The reaction mixture was ice-cooled, and water (72 mL) and 28% aqueous ammonia (60.4 g) were slowly added. To the obtained solution were added 1-butanol (200 mL) and 28% aqueous ammonia (3.0 g), and the aqueous layer was removed. To the aqueous layer was added 28% aqueous ammonia (4.5 g), and the mixture was reextracted with 1-butanol (100 mL) to give a solution of α-methylprolinamide in 1-butanol. This solution was concentrated to remove water, concentrated hydrochloric acid (9.24 g, 0.089 mol) and cyclohexane (50 mL) were added, and the mixture was subjected to azeotropic dehydration under normal pressure with heating under reflux by Dean-Stark apparatus. After cooling to room temperature, the crystals were collected by filtration, washed with 1-butanol and ethyl acetate and dried under reduced pressure to give α-methylprolinamide hydrochloride (11.3 g) as pale-yellow crystals. purity 75 wt% (HPLC analysis), 0.051 mol, yield 31%.

[Example 12]

Production of (S)-α-methylproline and (R)-α-methylprolinamide (in the above-mentioned formulas (4) and (5), $R^1$=Me, $R^2$=$R^3$=H; step (d) enzymatic resolution of racemate)

α-Methylprolinamide hydrochloride (32.2 g, purity 97%, 190 mmol) obtained according to the method of Example 7 and water (34.7 ml) were charged in a 500 ml Erlenmeyer flask, and the mixture was adjusted to pH 7.0 with 5% aqueous sodium hydroxide. To the mixture was added a solution of peptidase R (trade name, manufactured by Amano Enzyme Inc., derived from Rhizopus oryzae) (2.3 g) in water (15.5 ml) was added, and the mixture was reacted at 40, stirring number 250 rpm for 60 hr. As a result of purity analysis and optical purity analysis by HPLC analysis, the pure content of (S)-α-methylproline was 10.5 g (81.0 mmol, yield 342.7%) and the optical purity was 99.0%ee. To the reaction mixture were added water (50 ml) and activated carbon (15 g) and the mixture was shaken at 25°C for 1 hr. The activated carbon was removed by celite filtration, the obtained aqueous solution was passed through an ion exchange resin (Amberlyst (registered trademark) A-26(OH)), and (R)-α-methylprolinamide was eluted with water and (S)-α-methylproline was eluted with 1 M aqueous acetic acid. As a result of purity analysis and optical purity analysis by HPLC, the pure content of (R)-α-methylprolinamide was 12.6 g (98.5 mmol, yield 52%, 87.1%ee), and the pure content of (S)-α-methylproline was 11.3 g (87.6 mmol, yield 46%, 99.0%ee).

[Example 13]

Production of α-methylprolinamide (in the above-mentioned formula (3), $R^1$=Me, $R^2$=$R^3$=H; step (a) reaction in DMSO-water solvent, step (b) hydration with alkaline hydrogen peroxide water)

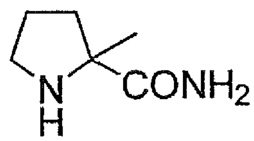

5-Chloro-2-pentanone (0.57 ml, 5.0 mmol), sodium cyanide (270 mg, 5.5 mmol), ammonium acetate (1.16 g, 15 mmol), water (2.3 ml) and DMSO (2.3 ml) were charged in a flask, and the mixture was reacted at 50°C for 1 hr. The reaction mixture was analyzed based on NMR. As a result, 2-cyano-2-methylpyrrolidine and 2-methyl-1-pyrroline were produced at 1:0.4 ratio.

To the reaction mixture were added sodium cyanide (250 mg, 5.0 mmol), 50 w/v% NaOH aqueous solution (1.0 ml, 13 mmol) and 35% hydrogen peroxide water (1.3 ml, 15 mmol), and the mixture was reacted at room temperature for 1 hr. Sodium thiosulfate (0.79 g, 5.0 mmol) was added, excess oxidizing agent was decomposed, and the mixture was extracted 2 times with ethyl acetate. As a result of HPLC analysis, α-methylprolinamide was contained in the organic layer (235 mg, 1.83 mmol, yield 37%) and in the aqueous layer (149 mg, 1.16 mmol, yield 23%).

[Example 14]

Production of α-methylprolinamide (in the above-mentioned formula (3), $R^1$=Me, $R^2$=$R^3$=H; step (b) hydration with alkaline hydrogen peroxide water)

A solution of 2-cyano-2-methylpyrrolidine obtained according to the method of Example 5 in t-butanol (0.58 g, 19 wt%, 1.0 mmol), sodium cyanide (49 mg, 1 mmol) and DMSO (0.4 ml) were charged in a flask. Under ice-cooling, 35% hydrogen peroxide water (0.13 ml, 1.5 mmol) was added, and the mixture was gradually warmed to room temperature. After 21 hr, sodium cyanide (49 mg, 1 mmol) and 35% hydrogen peroxide water (0.13 ml, 1.5 mmol) were added at 15°C, and the mixture was reacted for 3 hr. Under ice-cooling, sodium bisulfite (53 mg, 0.5 mmol) was added to decompose excess oxidizing agent, and the precipitate was filtered off. The filtrate was concentrated to give an orange oily substance (608 mg). By HPLC analysis, it contained α-methylprolinamide (94 mg, 0.73 mmol, yield 73%).

[Example 15]

Production of 1-(t-butoxycarbonyl)-2-cyano-2-methylpyrrolidine (in the above-mentioned formula (7), $R^1$=Me, $R^2$=Boc, $R^3$=H; step (a) reaction in t-butanol-water solvent, Boc protection)

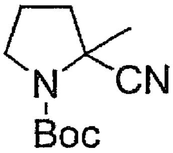

5-Chloro-2-pentanone (2.41 g, 20 mmol), sodium cyanide (1.08 g, 22 mmol), ammonium acetate (4.62 g, 60 mmol), water (10 ml) and t-butanol (10 ml) were charged in a flask, and the mixture was reacted at 50°C for 6 hr. To the reaction mixture were added ethyl acetate (10 ml) and 50 w/v% NaOH aqueous solution (2.4 ml), and the aqueous layer was removed. To the organic layer were added sodium cyanide (0.49 g, 10 mmol) and water (2 ml), di-t-butyl dicarbonate (6.55 g, 30 mmol) was added, and the mixture was reacted at room temperature for 5 hr, and at 50°C for 1 hr. At room temperature, the aqueous layer was removed, washed with saturated brine, and the organic layer was concentrated. The obtained residue was purified by silica gel column chromatography to give 1-(t-butoxycarbonyl)-2-cyano-2-methylpyrrolidine (2.38 g, 11.3 mmol, yield 57%) as a colorless oil.
$^1$H-NMR(400MHz,$CDCl_3$) $\delta$ 1.53(9H,brs), 1.70(3H,brs), 1.86-2.23(3H,m), 2.46-2.58(1H,m), 3.36-3.48(1H,m), 3.50-3.66(1H,m).

[Example 16]

Production of N-(t-butoxycarbonyl)-$\alpha$-methylprolinamide (in the above-mentioned formula (3), $R^1$=Me, $R^2$=Boc, $R^3$=H; step (b) hydration with alkaline hydrogen peroxide water)

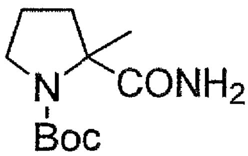

1-(t-Butoxycarbonyl)-2-cyano-2-methylpyrrolidine (1.88 g, 8.95 mmol) obtained in Example 15, 1 N NaOH aqueous solution (4.5 ml), methanol (9.4 ml) and 30% hydrogen peroxide water (0.91 ml, 9.0 mmol) were charged in a flask. The mixture was reacted at room temperature for 30 min, 30% hydrogen peroxide water (0.91 ml, 9.0 mmol) was added again, and the mixture was further reacted for 30 min. To the reaction mixture were added sodium bisulfite (0.95 g), ethyl acetate and saturated brine, and the aqueous layer was removed. The organic layer was dried over magnesium sulfate and concentrated. The obtained residue was purified by silica gel column chromatography to give N-(t-butoxycarbonyl)-$\alpha$-methylprolinamide (1.23 g, 5.39 mmol, yield 60%) as white crystals.
$^1$H-NMR(400MHz,$CDCl_3$,1:1 mixture of rotamers) $\delta$ 1.47(9H,brs), 1.55(1.5H,brs), 1.65(1.5H,brs), 1.60-2.05(3H,m), 2.24-2.36(0.5H,m), 2.57-2.69(0.5H,m), 3.36-3.66(2H,m), 5.23-5.41(1H,m), 5.82-5.98(0.5H,m), 7.12-7.28(0.5H,m).

[Reference Example 1]

Production of $\alpha$-methylprolinamide hydrochloride (in the above-mentioned formula (3), $R^1$=Me, $R^2$=$R^3$=H; removal of Boc group)

N-(t-Butoxycarbonyl)-$\alpha$-methylprolinamide (0.76 g, 3.33 mmol) obtained in Example 14, methanol (0.76 ml) and 4 N hydrochloric acid-ethyl acetate solution (1.7 ml) were added in a flask, and the mixture was stirred at room temperature for 3 hr, and at 40°C for 3 hr. The reaction mixture was ice-cooled, ethyl acetate (0.76 ml) was added, and the crystals were collected by filtration and dried under reduced pressure to give $\alpha$-methylprolinamide hydrochloride (0.50 g, 3.04 mmol, yield 91%) as white crystals.

[Example 17]

Production of 1-benzyl-2-cyano-2-methylpyrrolidine (in the above-mentioned formula (7), $R^1$=Me, $R^2$=Bn, $R^3$=H; step (a) using benzylamine and acetic acid, reaction in t-butanol-water solvent)

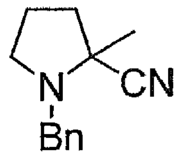

5-Chloro-2-pentanone (0.57 ml, 5.0 mmol), sodium cyanide (270 mg, 5.5 mmol), benzylamine (1.64 ml, 15 mmol), acetic acid (0.86 ml, 15 mmol), water (2.5 ml) and t-butanol (2.5 ml) were charged in a flask, and the mixture was reacted at 50°C for 2 hr. To the reaction mixture were added ethyl acetate (10 ml) and 50 w/v% NaOH aqueous solution (1.2 ml), and the aqueous layer was removed. The organic layer was concentrated to give a pale-brown oily substance (2.15 g). From the results of NMR analysis, this oily substance was a mixture containing 1-benzyl-2-cyano-2-methylpyrrolidine (47 wt%, 1.01 g, quantitatively), benzylamine (48 wt%), ethyl acetate (2 wt%) and t-butanol (3 wt%). $^1$H-NMR(400MHz,$CDCl_3$) $\delta$ 1.56(3H,s), 1.74-1.95(3H,m), 2.30-2.45(2H,m), 2.99(1H,ddd,J=9.8,8.3,3.3Hz), 3.35(1H,d,J=13.1Hz), 4.02(1H,d,J=13.1Hz), 7.24-7.37(5H,m).

[Example 18]

Production of 1-benzyl-2-cyano-2-methylpyrrolidine (in the above-mentioned formula (7), $R^1$=Me, $R^2$=Bn, $R^3$=H; step (a) reaction in ethyl acetate-water solvent)

5-Chloro-2-pentanone (2.28 ml, 20 mmol), sodium cyanide (1.08 g, 22 mmol), benzylamine (2.40 ml, 22 mmol), acetic acid (1.26 ml, 22 mmol), water (4.6 ml) and ethyl acetate (9.1 ml) were charged in a flask, and the mixture was reacted at 50°C for 3 hr. To the reaction mixture were added 50 w/v% NaOH aqueous solution (0.8 ml, 10 mmol) and sodium cyanide (0.50 g, 10 mmol), and the mixture was further reacted at 50°C for 2 hr. After cooling to room temperature, the aqueous layer was removed, and the organic layer was concentrated to give a pale-brown oily substance (4.30 g). From the results of NMR analysis, this oily substance was a mixture containing 1-benzyl-2-cyano-2-methylpyrrolidine (78 wt%, 3.35 g, yield 84%), 5-chloro-2-pentanone (2 wt%), chain intermediate 2-benzylamino-5-chloro-2-cyanopentane (12 wt%), benzylamine (6 wt%) and ethyl acetate (1 wt%).

[Example 19]

Production of 1-benzyl-2-cyano-2-methylpyrrolidine (in the above-mentioned formula (7), $R^1$=Me, $R^2$=Bn, $R^3$=H; step (a) reaction in toluene-water solvent)

5-Chloro-2-pentanone (0.57 ml, 5.0 mmol), sodium cyanide (270 mg, 5.5 mmol), benzylamine (0.60 ml, 5.5 mmol), acetic acid (0.32 ml, 5.5 mmol), water (1.1 ml) and toluene (2.3 ml) were charged in a flask, and the mixture was reacted at 50°C for 2 hr. To the reaction mixture was added sodium cyanide (0.17 g, 3.5 mmol), and the mixture was further reacted at 50°C for 4 hr. The reaction mixture was analyzed based on NMR. As a result, 5-chloro-2-pentanone (starting material), 1-benzyl-2-cyano-2-methylpyrrolidine (object product) and 2-benzylamino-5-chloro-2-cyanopentane (chain intermediate) were present at 0.04:1:0.27 ratio.

[Example 20]

Production of N-benzyl-$\alpha$-methylprolinamide (in the above-mentioned formula (3), $R^1$=Me, $R^2$=Bn, $R^3$=H; step (a) reaction in ethanol-water solvent, step (b) hydration with sulfuric acid)

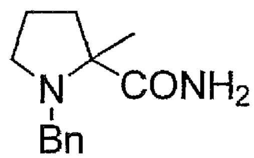

5-Chloro-2-pentanone (0.57 ml, 5.0 mmol), sodium cyanide (270 mg, 5.5 mmol), benzylamine (0.60 ml, 5.5 mmol), acetic acid (0.32 ml, 5.5 mmol), water (1.1 ml) and ethanol (1.1 ml) were charged in a flask, and the mixture was reacted at 40°C for 3 hr. To the reaction mixture was added benzylamine (0.16 ml, 1.5 mmol), and the mixture was further reacted at 40°C for 2 hr. The reaction mixture was analyzed based on NMR. As a result, 5-chloro-2-pentanone (starting material), 1-benzyl-2-cyano-2-methylpyrrolidine (object product), 2-benzylamino-5-chloro-2-cyanopentane (chain intermediate) and 5-chloro-2-cyano-2-pentanol (starting material cyanohydrin) were present at 0.02:1:0.02:0.08 ratio. To the reaction mixture was added 50 w/v% NaOH aqueous solution (0.4 ml, 5 mmol), and the mixture was extracted with toluene, and the organic layer was concentrated to give an orange oily substance (1.44 g).

Water (0.09 ml) and sulfuric acid (1.47 g, 15 mmol) were charged in another flask, the above-mentioned orange oily substance (1.44 g) was added under ice-cooling, and the mixture was washed with toluene. The mixture was reacted at 60°C for 3 hr, and water (0.57 ml) and 28% aqueous ammonia (2.4 ml) were slowly added under ice-cooling. The mixture was extracted with ethyl acetate, and the organic layer was concentrated to give an orange oily substance (2.00 g). As a result of HPLC analysis, N-benzyl-α-methylprolinamide (770 mg, 3.52 mmol, yield 70%) was contained.
$^1$H-NMR(400MHz,$CDCl_3$) δ 1.34(3H,s), 1.68-1.89(3H,m), 2.13-2.22(1H,m), 2.40(1H,td,J=9.1,7.3Hz), 2.97-3.03(1H,m), 3.35(1H,d,J=13.1Hz), 3.88(1H,d,J=13.1Hz), 5.36(1H,brs), 7.24-7.37(5H,m), 7.55(1H,brs).

[Example 21]

Production of N-benzyl-α-methylprolinamide (in the above-mentioned formula (3), $R^1$=Me, $R^2$=Bn, $R^3$=H; step (a) reaction in water solvent, step (b) hydration with sulfuric acid)

5-Chloro-2-pentanone (6.03 g, 50 mmol), sodium cyanide (2.7 g, 55 mmol), benzylamine (6.0 ml, 55 mmol), acetic acid (3.2 ml, 55 mmol) and water (11.4 ml) were charged in a flask, and the mixture was reacted at 40°C for 3 hr. To the reaction mixture was added 50 w/v% NaOH aqueous solution (4.0 ml, 50 mmol), and the mixture was further reacted at 60°C for 1 hr. The reaction mixture was analyzed based on NMR. As a result, it was 1-benzyl-2-cyano-2-methylpyrrolidine which was almost the object product. The aqueous layer was separated to give a pale-brown oily substance.

Sulfuric acid (14.7 g, 150 mmol) was charged in another flask, the above-mentioned pale-brown oily substance was added under ice-cooling and the mixture was washed with toluene. The mixture was reacted at 60°C for 7 hr, and at 70°C for 2 hr, water (50 ml), toluene (6 ml) and 50 w/v% NaOH aqueous solution (32 ml) were slowly added in a water bath to adjust pH to 10. The resulting crystals were collected by filtration, washed with water, and dried under reduced pressure to give N-benzyl-α-methylprolinamide (8.67 g) as a pale-brown solid. purity 98 wt% (HPLC analysis), 39.0 mmol, yield 78%.

[Example 22]

Production of 1-benzyl-2-cyano-2-methylpyrrolidine (in the above-mentioned formula (7), $R^1$=Me, $R^2$=Bn, $R^3$=H; step (a) without addition of acetic acid, reaction in ethanol-water solvent)

5-Chloro-2-pentanone (0.57 ml, 5.0 mmol), sodium cyanide (270 mg, 5.5 mmol), benzylamine (0.60 ml, 5.5 mmol), water (1.1 ml) and ethanol (1.1 ml) were charged in a flask, and the mixture was reacted at 50°C for 1.5 hr. The reaction mixture was analyzed based on NMR. As a result, 5-chloro-2-pentanone (starting material), 1-benzyl-2-cyano-2-methylpyrrolidine (object product) and cyclopentyl methyl ketone by-produced from the starting material were present at 0.02:1:0.32 ratio. As compared to Example 20 in which acetic acid was added, cyclopentyl methyl ketone was by-produced since the inside of the system became strongly basic; however, the object product 1-benzyl-2-cyano-2-methylpyrrolidine was obtained as a major product.

[Example 23]

Production of 1-benzyl-2-cyano-2-methylpyrrolidine (in the above-mentioned formula (7), $R^1$=Me, $R^2$=Bn, $R^3$=H; step (a) without addition of acetic acid, reaction in ethyl acetate-water solvent)

5-Chloro-2-pentanone (0.57 ml, 5.0 mmol), sodium cyanide (270 mg, 5.5 mmol), benzylamine (0.60 ml, 5.5 mmol), water (1.1 ml) and ethyl acetate (2.3 ml) were charged in a flask, and the mixture was reacted at 50°C for 4.5 hr. The reaction mixture was analyzed based on NMR. As a result, 5-chloro-2-pentanone (starting material), 1-benzyl-2-cyano-2-methylpyrrolidine (object product) and 2-benzylamino-5-chloro-2-cyanopentane (chain intermediate) were present at 0.02:1:0.06 ratio, and cyclopentyl methyl ketone was not observed. In the same manner as in Example 22, acid was not added; however, it is considered that ethyl acetate underwent hydrolysis to produce acetic acid, the inside of the system did not become strongly basic, and by-production of cyclopentyl methyl ketone was suppressed.

[Reference Example 2]

Production of α-methylprolinamide hydrochloride (in the above-mentioned formula (3), $R^1$=Me, $R^2$=$R^3$=H; removal of Bn group)

N-Benzyl-α-methylprolinamide (6.62 g, 30.3 mmol) obtained in Example 20, ethanol (33 ml) and 10% palladium carbon (PE-type manufactured by N.E. CHEMCAT CORPORATION, 55% water) (1.43 g, 0.61 mmol) were added into a flask. The mixture was reacted under normal pressure and a hydrogen atmosphere at room temperature for 2 hr, and at 40°C for 4 hr. The reaction mixture was filtered through celite, and the filtrate was concentrated to give α-methylprolinamide (4.12 g) as a pale-yellow oily substance. purity 92 wt% (HPLC analysis), 29.5 mmol, yield 97%.

The above-mentioned yellow oily substance (2.38 g, 17.1 mmol), ethanol (2 ml) and ethyl acetate (5 ml) were charged in a flask. 4 N Hydrochloric acid-ethyl acetate solution (4.5 ml) was added in a water bath, and the resulting crystals were collected by filtration, washed with ethyl acetate, and dried under reduced pressure to give α-methylprolinamide hydrochloride (2.42 g, 14.7 mmol, yield 86%) as white crystals.

[Reference Example 3]

Production of α-methylprolinamide (in the above-mentioned formula (3), $R^1$=Me, $R^2$=$R^3$=H; removal of Bn group)

N-Benzyl-α-methylprolinamide (1.01 g, 4.6 mmol) obtained in Example 20, acetic acid (0.53 ml, 9.2 mmol), methanol (5 ml) and 10% palladium carbon (PE-type manufactured by N.E. CHEMCAT CORPORATION, 55% water) (54 mg, 0.023 mmol) were added into a flask. The mixture was reacted at 60°C for 2 hr under normal pressure and a hydrogen atmosphere. The reaction mixture was filtered through celite and the filtrate was concentrated to give a pale-yellow oily substance (1.20 g). As a result of HPLC analysis, α-methylprolinamide (0.60 g, yield quantitatively) was contained.

[Example 24]

Production of (2R,1'R) and (2S,1'R)-1-(1'-phenylethyl)-2-cyano-2-methylpyrrolidine (in the above-mentioned formula (7), $R^1$=Me, $R^2$=(R)-1-phenylethyl, $R^3$=H; step (a) using (R)-α-methylbenzylamine, reaction in water solvent)

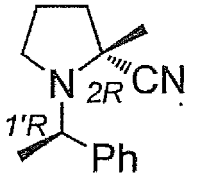

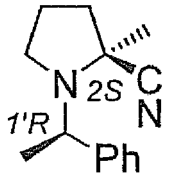

5-Chloro-2-pentanone (1.14 ml, 10 mmol), sodium cyanide (0.54 g, 11 mmol), (R)-α-methylbenzylamine (1.40 ml, 11 mmol), acetic acid (0.63 ml, 11 mmol) and water (2.3 ml) were charged in a flask, and the mixture was reacted at 40°C for 3 hr. To the reaction mixture was added 50 w/v% NaOH aqueous solution (0.80 ml, 10 mmol), and the mixture was further reacted at 60°C for 1 hr. After cooling to room temperature, the mixture was extracted with ethyl acetate, and the organic layer was dried over magnesium sulfate and concentrated to give a pale-brown oily substance (2.35 g).

As a result of NMR analysis, this oily substance was a mixture containing (2R,1'R) and (2S,1'R)-1-(1'-phenylethyl)-2-cyano-2-methylpyrrolidine (83 wt%, 1.95 g, yield 91%), 5-chloro-2-pentanone (2 wt%), (R)-α-methylbenzylamine (11 wt%) and ethyl acetate (4 wt%), and the diastereomer ratio was (2R,1'R):(2S,1'R)=1:0.4. The stereochemistry at the 2-position was determined after derivatization to optically active α-methylproline (see Reference Example 5).
(2R,1'R)-1-(1'-phenylethyl)-2-cyano-2-methylpyrrolidine $^1$H-NMR(400MHz,$CDCl_3$) δ 1.02(3H,s), 1.48(3H,d,J=6.8Hz), 1.79-1.92(3H,m), 2.22-2.32(1H,m), 2.75-2.83(1H,m), 3.18-3.25(1H,m), 3.92(1H,q,J=6.8Hz), 7.21-7.39(5H,m).
(2S,1'R)-1-(1'-phenylethyl)-2-cyano-2-methylpyrrolidine $^1$H-NMR(400MHz,$CDCl_3$) δ 1.48(3H,d,J=6.8Hz), 1.67(3H,s), 1.69-1.78(2H,m), 1.92-1.99(1H,m), 2.32-2.39(1H,m), 2.47(1H,dt,J=9.6,8.1Hz), 2.84-2.91(1H,m), 3.84(1H,q,J=6.8Hz), 7.21-7.39(5H,m).

[Example 25]

Production of (2R,1'R) and (2S,1'R)-N-(1'-phenylethyl)-α-methylprolinamide (in the above-mentioned formula (5), $R^1$=Me, $R^2$=(R)-1-phenylethyl, $R^3$=H; step (a) using (R)-α-methylbenzylamine, reaction in water solvent, step (b) hydration with sulfuric acid, step (f) separation by silica gel column chromatography)

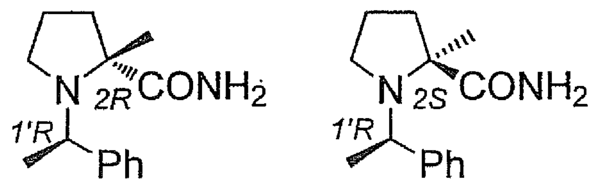

5-Chloro-2-pentanone (1.25 ml, 11 mmol), sodium cyanide (0.54 g, 11 mmol), (R)-α-methylbenzylamine (1.28 ml, 10 mmol), acetic acid (0.63 ml, 11 mmol) and water (2.0 ml) were charged in a flask, and the mixture was reacted at 40°C for 3 hr. To the reaction mixture was added 50 w/v% NaOH aqueous solution (0.80 ml, 10 mmol), and the mixture was further reacted at 60°C for 2 hr. After cooling to room temperature, the aqueous layer was separated to give a pale-brown oily substance.
Sulfuric acid (3.9 g, 40 mmol) was charged in another flask, the above-mentioned pale-brown oily substance was added in a water bath and the mixture was washed with toluene. The mixture was reacted at 60°C for 6 hr, and water (2 ml) and 50 w/v% NaOH aqueous solution (6.4 ml) were slowly added in a water bath to adjust pH to 10. The mixture was extracted with ethyl acetate, and the organic layer was dried over magnesium sulfate, and concentrated. The residue was purified by silica gel column chromatography to give two fractions of (2R,1'R) and (2S,1'R)-N-(1'-phenylethyl)-α-methylprolinamide. The stereochemistry of the 2-position was determined after derivatization to optically active α-methylproline (see Reference Example 5).

Fraction 1: (2R,1'R):(2S,1'R)=96:4, 0.48 g, purity 85%(NMR), 1.75 mmol, yield 18%.
Fraction 2: (2R,1'R):(2S,1'R)=60:40, 1.52 g, purity 88%(NMR), 5.80 mmol, yield 58%.

(2R,1'R)-N-(1'-phenylethyl)-α-methylprolinamide $^1$H-NMR(400MHz,$CDCl_3$) δ 1.30(3H,d,J=6.6Hz), 1.37(3H,s), 1.64-1.73(2H,m), 1.80-1.88(1H,m), 2.16-2.32(2H,m), 2.69-2.75(1H,m), 3.62(1H,q,J=6.6Hz), 5.45(1H,brs), 7.22-7.28(1H,m), 7.28-7.35(4H,m), 7.45(1H,brs).
(2S,1'R)-N-(1'-phenylethyl)-α-methylprolinamide $^1$H-NMR(400MHz,$CDCl_3$) δ 1.41(3H,s), 1.42(3H,d,J=6.8Hz), 1.66-1.89(3H,m), 2.12-2.22(1H,m), 2.90-3.00(1H,m), 3.09-3.15(1H,m), 4.04(1H,q,J=6.8Hz), 5.17(1H,brs), 7.02(1H,brs), 7.20-7.27(1H,m), 7.29-7.35(4H,m).

[Reference Example 4]

Production of (R)-α-methylprolinamide (in the above-mentioned formula (5), $R^1$=Me, $R^2$=$R^3$=H; removal of 1-phenylethyl group)

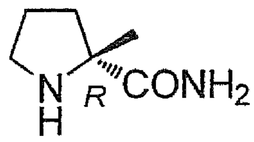

(2R,1'R)-N-(1'-Phenylethyl)-α-methylprolinamide (0.48 g, 1.75 mmol, diastereomer ratio 96:4) obtained in Example 25, acetic acid (0.11 ml, 1.9 mmol), methanol (2 ml) and 10% palladium carbon (PE-type manufactured by N.E. CHEMCAT CORPORATION, 55% water) (21 mg, 0.009 mmol) were added into a flask. The mixture was reacted at 60°C for 2 hr under normal pressure and a hydrogen atmosphere. The reaction mixture was filtered through celite, and the filtrate was concentrated to give (R)-α-methylprolinamide (0.40 g) as a colorless oil. purity 58 wt% (NMR analysis), quantitatively.

[Reference Example 5]

Production of (R)-α-methylproline (in the above-mentioned formula (4), $R^1$=Me, $R^2$=$R^3$=H; amide hydrolysis, determination of absolute configuration of α-methylproline)

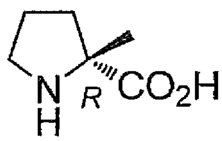

(R)-α-Methylprolinamide (0.40 g, purity 58%, 1.78 mmol) obtained in Reference Example 4 and 6 M hydrochloric acid (2 ml) were charged in a flask, and the mixture was reacted at 90°C for 4 hr. As a result of HPLC analysis, it contained (R)-α-methylproline (204 mg, 1.58 mmol, yield 90%) with optical purity 89%ee.

[Example 26]

Production of (2S,1'S) and (2R,1'S)-N-(1'-phenylethyl)-α-methylprolinamide (in the above-mentioned formula (3), $R^1$=Me, $R^2$=(S)-1-phenylethyl, $R^3$=H; step (a) using (S)-α-methylbenzylamine, reaction in water solvent, step (b) hydration with sulfuric acid)

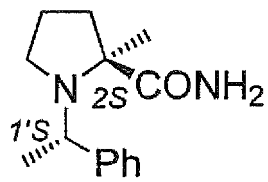

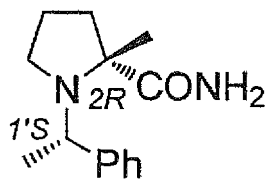

5-Chloro-2-pentanone (13.3 g, 110 mmol), sodium cyanide (5.4 g, 110 mmol), (S)-α-methylbenzylamine (12.1 g, 100 mmol), acetic acid (6.3 ml, 110 mmol) and water (24 ml) were charged in a flask, and the mixture was reacted at 40°C for 3 hr. To the reaction mixture was added 50 w/v% NaOH aqueous solution (8.0 ml, 100 mmol), and the mixture was further reacted at 60°C for 2 hr. After cooling to room temperature, the aqueous layer was separated to give a pale-brown oily substance.

Sulfuric acid (39 g, 400 mmol) was charged in another flask, the above-mentioned pale-brown oily substance was added in a water bath, and the mixture was washed with toluene. The mixture was reacted at 60°C for 3 hr, and at 70°C for 3 hr, and water (36 ml), 28% aqueous ammonia (60 ml) and ethyl acetate were slowly added in a water bath to adjust pH to 9. The mixture was extracted with ethyl acetate, and the organic layer was washed 3 times with water and once with saturated brine and concentrated to give (2S,1'S) and (2R,1'S)-N-(1'-phenylethyl)-α-methylprolinamide (23.8 g, purity 80 wt% (HPLC analysis), 81.4 mmol, yield 81%, (2S,1'S):(2R,1'S)=1:0.4) as a pale-brown oily substance.

[Example 27]

Production of (2S,1'S) and (2R,1'S)-N-(1'-phenylethyl)-α-methylprolinamide (in the above-mentioned formula (5), $R^1$=Me, $R^2$=(S)-1-phenylethyl, $R^3$=H; step (f) separation by silica gel column chromatography)

(2S,1'S) and (2R,1'S)-N-(1'-Phenylethyl)-α-methylprolinamide (pure amount 9.80 g, 42.2 mmol) obtained according to the method of Example 26 and containing acetic acid (not less than 1 equivalent) was purified by column chromatography using silica gel (100 g, silica gel 60N (spherical, neutral) 63-210 μm manufactured by KANTO CHEMICAL CO., INC.) to give 2 fractions of (2S,1'S) and (2R,1'S)-N-(1'-phenylethyl)-α-methylprolinamide. eluent: hexane:ethyl acetate=2:1 - 1:1.

Fraction 1: 6.71 g. As a result of NMR analysis, (2S,1'S):(2R,1'S)=100:0, purity 72%, 20.8 mmol, yield 49%. containing acetic acid (20 wt%) and ethyl acetate (8 wt%).
Fraction 2: 3.11 g. As a result of NMR analysis, (2R,1'S):(2S,1'S)=87:13, purity 82%, 11.0 mmol, yield 25%. containing acetic acid (13 wt%) and ethyl acetate (5 wt%).

[Reference Example 6]

Production of α-methylprolinamide hydrochloride (in the above-mentioned formula (3), $R^1$=Me, $R^2$=$R^3$=H; removal of 1-phenylethyl group, formation of hydrochloride)

(2S,1'S) and (2R,1'S)-N-(1'-Phenylethyl)-α-methylprolinamide (5.94 g, purity 80%, 20.4 mmol) obtained in Example 26, ethyl acetate (25 ml) and activated carbon (2.5 g) were charged in a flask, and the mixture was stirred at room temperature for 1 hr to allow for adsorption of colored components and impurity. After celite filtration, the filtrate was concentrated, and methanol (24 ml), acetic acid (1.4 ml, 25 mmol) and 10% palladium carbon (PE-type manufactured by N.E. CHEMCAT CORPORATION, 55% water) (0.24 g, 0.10 mmol) were added. The mixture was reacted under normal pressure and a hydrogen atmosphere at 60°C for 4.5 hr, and filtered through celite, and the filtrate was concentrated to give a pale-yellow oily substance (5.14 g).
The above-mentioned yellow oily substance, methanol (1.6 ml) and ethyl acetate (6 ml) were charged in a flask. 4 N Hydrochloric acid-ethyl acetate solution (6 ml) was added in a water bath, and the resulting crystals were collected by filtration, washed with ethyl acetate, and dried under reduced pressure to give α-methylprolinamide hydrochloride (2.46 g, 14.9 mmol, yield 73%) as white crystals.

[Example 28]

Production of (S)-α-methylproline and (R)-α-methylprolinamide (in the above-mentioned formulas (4) and (5), $R^1$=Me, $R^2$=$R^3$=H; step (d) enzymatic resolution of α-methylprolinamide ((S) : (R)=7:3))

α-Methylprolinamide hydrochloride (2.0 g, 12.2 mmol) obtained in Reference Example 6 and water (2.0 ml) were charged in a 50 ml sample tube, and adjusted to pH 7.0 with 5% aqueous sodium hydroxide. A solution of peptidase R (trade name, Amano Enzyme Inc., derived from Rhizopus oryzae) (0.15 g) in water (0.9 ml) was added to the mixture, and the mixture was reacted at 40°C, stirring number 250 rpm for 162 hr. To the reaction mixture were added water (2.5 ml) and activated carbon (0.75 g), and the mixture was shaken at 25°C for 1 hr. The activated carbon was removed by celite filtration, the obtained aqueous solution was passed through an ion exchange resin (DIAION (registered trademark) PA312LOH), and (R)-α-methylprolinamide was eluted with water, and (S)-α-methylproline was eluted with 1 M aqueous acetic acid. As a result of purity analysis and optical purity analysis by HPLC, (R)-α-methylprolinamide had a pure content of 454 mg (3.54 mmol, yield 29%, 93.5%ee), and (S)-α-methylproline had a pure content of 799 mg (6.19 mmol, yield 51%, 99.1%ee).

[Example 29]

Production of (2S,1'R)-N-(1'-Phenylethyl)-a-methylprolinamide D-tartrate (in the above-mentioned formula (5), $R^1$=Me, $R^2$=(R)-1-phenylethyl, $R^3$=H; step (e) resolution of diastereomeric salts by D-tartaric acid)

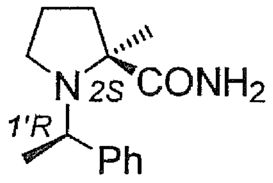
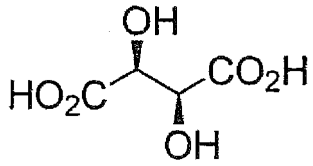

(2R,1'R) and (2S,1'R)-N-(1'-Phenylethyl)-α-methylprolinamide (48.4 mg, 0.21 mmol, (2R,1'R):(2S,1'R)=60:40) obtained in Example 25, D-tartaric acid (31.3 mg, 0.21 mmol), ethyl acetate (0.2 ml) and methanol (0.1 ml) were added into a vial, and the mixture was dissolved by heating to 50°C. The mixture was cooled to room temperature, and the resulting crystals were collected by filtration, and dried under reduced pressure to give white crystals (18.9 mg). As a result of HPLC and NMR analyses, the obtained crystals were 1:1 salt (0.049 mmol, yield 24%, (2S,1'R):(2R,1'R)=20:1) of (2S,1'R)-N-(1'-phenylethyl)-α-methylprolinamide and D-tartaric acid. $^1$H-NMR(400MHz, DMSO-d6) δ 1.28(3H,s), 1.35(3H,d,J=6.8Hz), 1.62-1.79(3H,m), 1.95-2.03(1H,m), 2.83-2.91(1H,m), 3.05-3.12(1H,m), 3.99-4.06(1H,m), 4.26(2H,s), 6.96(2H,brs), 7.19-7.24(1H,m), 7.28-7.34(2H,m), 7.35-7.39(2H,m).

[Example 30]

Production of (2R,1'S)-N-(1'-phenylethyl)-α-methylprolinamide L-tartrate and (2S,1'S)-N-(1'-phenylethyl)-α-methylprolinamide (in the above-mentioned formula (5), $R^1$=Me, $R^2$=(S)-1-phenylethyl, $R^3$=H; step (e) resolution of diastereomeric salts by L-tartaric acid)

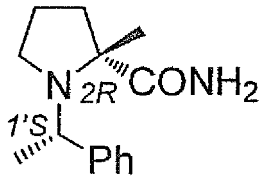
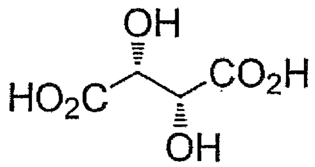
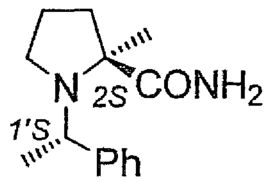

L-Tartaric acid (0.18 g, 1.2 mmol) and methanol (0.6 ml) were charged in a flask, and the mixture was dissolved by heating to 60°C. To this mixture were added a solution of (2S,1'S) and (2R,1'S)-N-(1'-phenylethyl)-α-methylprolinamide obtained according to the method of Example 26 in ethyl acetate (2.24 g, purity 32 wt%, 3.0 mmol, (2S,1'S):(2R,1'S)=7:3), methanol (0.4 ml) and ethyl acetate (3 ml), and the mixture was gradually cooled to ice-cooling. The resulting crystals were collected by filtration, washed with ethyl acetate, and dried under reduced pressure to give (2R,1'S)-N-(1'-phenylethyl)-α-methylprolinamide L-tartrate (314 mg, 0.82 mmol, yield 27%, (2R,1'S):(2S,1'S)=94:6) as white crystals. The filtrate was concentrated, ethyl acetate and saturated aqueous sodium hydrogen carbonate solution were added, the aqueous layer was removed, and the organic layer was dried over magnesium sulfate, and concentrated to give (2S,1'S)-N-(1'-phenylethyl)-α-methylprolinamide (683 mg, HPLC purity analysis: purity 77%, 2.26 mmol, yield 73%, (2S,1'S):(2R,1'S)=95:5) as a pale-yellow oily substance.

[Example 31]

Production of (2S,1'S)-N-(1'-phenylethyl)-α-methylprolinamide (S)-mandelate (in the above-mentioned formula (5), $R^1$=Me, $R^2$=(S)-1-phenylethyl, $R^3$=H; step (e) obtainment of (S)-mandelate seed crystals)

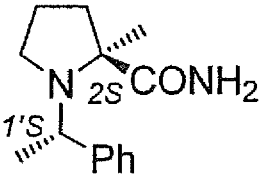
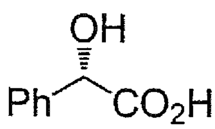

(2S,1'S)-N-(1'-Phenylethyl)-α-methylprolinamide (50 mg, purity 72%, 0.16 mmol, (2S,1'S):(2R,1'S)=100:0) obtained in Example 27, (S)-mandelic acid (32.7 mg, 0.22 mmol) and ethyl acetate (0.2 ml) were added into a vial, and

the mixture was dissolved by heating. The mixture was left standing at room temperature in an open system to volatilize ethyl acetate. After 2 weeks, the resulting crystals were suspended in ethyl acetate, filtered, and dried under reduced pressure to give white crystals (41.0 mg). As a result of HPLC and NMR analyses, the obtained crystals were (2S,1'S)-N-(1'-phenylethyl)-α-methylprolinamide (S)-mandelate (1:1). 0.11 mmol, yield 69%. $^1$H-NMR(400MHz,acetone-d6) δ 1.28(3H,s), 1.28(3H,d,J=6.6Hz), 1.65-1.81(3H,m), 2.07-2.18(1H,m), 2.29(1H,q,J=8.5Hz), 2.68-2.75(1H,m), 3.66(1H,q,J=6.6Hz), 5.20(1H,s), 7.20-7.25(1H,m), 7.27-7.34(3H,m), 7.34-7.39(2H,m), 7.42-7.47(2H,m), 7.49-7.53 (2H,m) .

[Example 32]

Production of (2S,1'S)-N-(1'-phenylethyl)-α-methylprolinamide (S)-mandelate (in the above-mentioned formula (5), $R^1$=Me, $R^2$=(S)-1-phenylethyl, $R^3$=H; step (e) resolution of diastereomeric salts by (S)-mandelic acid)

A solution of (2S,1'S) and (2R,1'S)-N-(1'-phenylethyl)-α-methylprolinamide obtained according to the method of Example 26 in ethyl acetate (2.23 g, purity 32 wt%, 3.0 mmol, (2S,1'S):(2R,1'S)=7:3) and (S)-mandelic acid (456 mg, 3.0 mmol) were added to a flask, and the mixture was dissolved by heating at 40°C. After cooling to 30°C, a trace amount of the seed crystals of (2S,1'S)-N-(1'-phenylethyl)-α-methylprolinamide (S)-mandelate obtained according to the method of Example 31 was added. As a result, the crystals precipitated. After cooling to 20°C, the crystals were collected by filtration, washed with ethyl acetate, and dried under reduced pressure to give (2S,1'S)-N-(1'-phenylethyl)-α-methylprolinamide (S)-mandelate (753 mg, 1.96 mmol, yield 64%, 94.2%de) as white crystals.

[Example 33]

Production of (2S,1'S)-N-(1'-phenylethyl)-α-methylprolinamide (S)-mandelate (in the above-mentioned formula (5), $R^1$=Me, $R^2$=(S)-1-phenylethyl, $R^3$=H; step (e) resolution of diastereomeric salts by (S)-mandelic acid)

A solution of (2S,1'S) and (2R,1'S)-N-(1'-phenylethyl)-α-methylprolinamide obtained according to the method of Example 26 in ethyl acetate (7.26 g, purity 32 wt%, 10.0 mmol, (2S,1'S):(2R,1'S)=7:3) and (S)-mandelic acid (1.22 g, 8.0 mmol) were added into a flask, and the mixture was dissolved by heating at 40°C. A trace amount of the seed crystals of (2S,1'S)-N-(1'-phenylethyl)-α-methylprolinamide (S)-mandelate obtained according to the method of Example 31 was added at 40°C. As a result, the crystals precipitated. After gradually cooling to ice-cooling, the crystals were collected by filtration, washed with ethyl acetate, and dried under reduced pressure to give (2S,1'S)-N-(1'-phenylethyl)-α-methylprolinamide (S)-mandelate (2.36 g, 6.13 mmol, yield 61%, 96.6%de) as white crystals.

[Reference Example 7]

Production of (S)-α-methylproline (in the above-mentioned formula (4), $R^1$=Me, $R^2$=$R^3$=H; removal of 1-phenylethyl group, amide hydrolysis)

(2S,1'S)-N-(1'-Phenylethyl)-α-methylprolinamide (S)-mandelate (2.36 g, 6.13 mmol, 96.6%de) obtained in Example 33, 1-butanol (7 ml), 50 w/v% aqueous NaOH (0.54 ml), water (2.8 ml) and saturated brine (1 ml) were added to a flask, and the mixture was stirred. The aqueous layer was removed, and the organic layer was washed with saturated brine (1 ml) to remove (S)-mandelic acid. To the obtained organic layer were added acetic acid (0.39 ml, 6.7 mmol) and 10% palladium carbon (PE-type manufactured by N.E. CHEMCAT CORPORATION, 55% water) (72 mg, 0.031 mmol), and the mixture was reacted under normal pressure and a hydrogen atmosphere at 60°C for 2 hr. The reaction mixture was filtered through celite to give a solution of (S)-α-methylprolinamide in 1-butanol. To this solution were added 50 w/v% aqueous NaOH (0.59 ml) and water (0.59 ml), the aqueous layer was separated and acetic acid was removed. To the organic layer were added water (1 ml) and sulfuric acid (0.49 ml, 9.2 mmol) to extract (S)-α-methylprolinamide in an aqueous layer, and the organic layer was removed. Furthermore, sulfuric acid (0.16 ml, 3.1 mmol) was added, and the mixture was refluxed for 13 hr to perform amide hydrolysis. After cooling to room temperature, 50 w/v% aqueous NaOH (1.47 ml) was added, the resulting slurry was concentrated under reduced pressure, and ammonia produced by hydrolysis was removed. The pH was adjusted to about 8 with sulfuric acid and 50 w/v% aqueous NaOH, and acetone (5 ml) was added to precipitate sodium sulfate. The resulting crystals were filtered off, and the filtrate was concentrated. 1-Butanol and cyclohexane were added, and the mixture was subjected to azeotropic dehydration under normal pressure with heating under reflux by Dean-Stark apparatus. After cooling to room temperature, the resulting crystals were collected by filtration, washed with ethyl acetate, and dried under reduced pressure to give (S)-α-methylproline (531 mg, HPLC purity analysis: 93 wt%, 3.84 mmol, yield 63%) as white crystals. The optical purity of (S)-α-methylproline was 99.8%ee.

[Example 34]

Separation of (2S,1'S) and (2R,1'S)-N-(1'-phenylethyl)-α-methylprolinamide (step (f) separation by reversed-phase HPLC)

(2S,1'S) and (2R,1'S)-N-(1'-Phenylethyl)-α-methylprolinamide ((2S,1'S):(2R,1'S)=7:3) obtained in Example 26 was analyzed under the following reversed-phase HPLC conditions. As a result, a large difference was found in the retention time. This means that reversed-phase column preparative chromatography including simulated moving bed chromatography can be efficiently applied.
reversed-phase HPLC conditions

column: L-column (4.6 mm × 250 mm) manufacture by Chemicals Evaluation and Research Institute, Japan, mobile phase: 20 mM acetic acid-20 mM ammonium acetate aqueous
solution/methanol=40/60, flow rate: 1.0 ml/min, column temperature: 40°C, UV: 220 nm, retention time: (2R,1'S)-N-(1'-phenylethyl)-α-methylprolinamide 5.8 min, (2S,1'S)-N-(1'-phenylethyl)-α-methylprolinamide 9.3 min

[Example 35]

Separation of (2S,1'S) and (2R,1'S)-N-(1'-phenylethyl)-α-methylprolinamide by synthetic adsorbent column chromatography (step (f) separation by synthetic adsorbent column chromatography)

(2S,1'S) and (2R,1'S)-N-(1'-Phenylethyl)-α-methylprolinamide ((2S,1'S):(2R,1'S)=7:3) obtained in Example 26 was analyzed under the following HPLC conditions using synthetic adsorbent column. As a result, a large difference was found in the retention time. This means that reversed-phase column preparative chromatography including simulated moving bed chromatography can be efficiently applied.
HPLC conditions using synthetic adsorbent column

column: MCI (registered trademark)-GEL CHP10M (4.6 mm × 150 mm) manufactured by Mitsubishi Chemical Corporation, mobile phase: 20 mM acetic acid-20 mM ammonium acetate aqueous solution/acetonitrile=50/50, flow rate: 1.0 ml/min, column temperature: 40°C, UV: 220 nm, retention time: (2R,1'S)-N-(1'-phenylethyl)-α-methylprolinamide 3.7 min, (2S,1'S)-N-(1'-phenylethyl)-α-methylprolinamide 5.1 min

[Example 36]

Production of 1-(carbamoylphenylmethyl)-2-cyano-2-methylpyrrolidine (in the above-mentioned formula (7), $R^1$=Me, $R^2$=carbamoylphenylmethyl, $R^3$=H; step (a) using D-phenylglycinamide hydrochloride, reaction in ethyl acetate-water solvent)

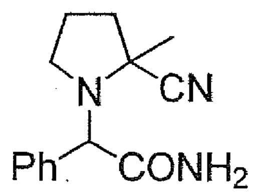

5-Chloro-2-pentanone (0.60 g, 5 mmol), sodium cyanide (0.37 g, 7.5 mmol), D-phenylglycinamide hydrochloride (0.93 g, 5 mmol), acetic acid (0.43 ml, 7.5 mmol), 50 w/v% NaOH aqueous solution (0.4 ml, 5 mmol), water (1.2 ml) and ethyl acetate (2.4 ml) were charged in a flask, and the mixture was reacted at 40°C for 2 hr and at 50°C for 2 hr. To the reaction mixture was added 50 w/v% NaOH aqueous solution (0.2 ml, 2.5 mmol), and the mixture was further reacted at 60°C for 2 hr, and cooled to room temperature. To the reaction mixture were added 50 w/v% NaOH aqueous solution (0.2 ml, 2.5 mmol) and acetic acid (0.086 ml, 1.5 mmol), and the crystals were collected by filtration, washed with water and ethyl acetate, and dried under reduced pressure to give 1-(carbamoylphenylmethyl)-2-cyano-2-methylpyrrolidine (0.96 g, 3.9 mmol, yield 79%) as white crystals. Since derivatization to α-methylproline gave a racemate, it is considered that epimerization of the carbamoylphenylmethyl group proceeded during the reaction (see Reference Example 8).
7:3 diastereomeric mixture was observed by $^1$H-NMR, major product: $^1$H-NMR(400MHz,$CDCl_3$) δ 1.62(3H,s),

1.74-1.99(3H,m), 2.29-2.41(2H,m), 2.78-2.84(1H,m), 4.51(1H,s), 5.81(1H,brs), 6.97(1H,brs), 7.31-7.44(5H,m).
minor product: $^1$H-NMR(400MHz,$CDCl_3$) δ 0.85(3H,s), 1.74-1.99(3H,m), 2.29-2.41(1H,m), 2.70-2.78(1H,m), 3.43-3.50(1H,m), 4.19(1H,s), 5.58(1H,brs), 6.15(1H,brs), 7.31-7.44(3H,m), 7.52-7.58(2H,m).

[Example 37]

Production of N-(carbamoylphenylmethyl)-α-methylprolinamide (in the above-mentioned formula (3), $R^1$=Me, $R^2$=carbamoylphenylmethyl, $R^3$=H; step (b) hydration with sulfuric acid)

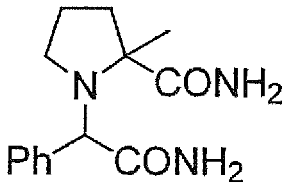

1-(Carbamoylphenylmethyl)-2-cyano-2-methylpyrrolidine (0.30 g, 1.23 mmol) obtained in Example 36 and sulfuric acid (1.0 g, 10 mmol) were charged in a flask, and the mixture was reacted at 50°C for 2 hr, and water and 28% aqueous ammonia were added in a water bath. The mixture was extracted with ethyl acetate, and the organic layer was dried over magnesium sulfate and concentrated to give a crude product (0.50 g) of N-(carbamoylphenylmethyl)-α-methylprolinamide as a colorless oil (NMR analysis: diastereomer ratio >5:1).
$^1$H-NMR (400MHz, $CDCl_3$) δ 1.45(3H, s), 1.63-1.72(1H, m), 1.80-1.94(2H, m), 2.19-2.27(1H,m) , 2.99-3.05(1H,m), 3.42-3.49(1H,m), 4.48(1H,s), 5.31(1H,brs), 5.38(1H,brs), 5.58(1H,brs), 6.97(1H,brs), 7.29-7.47(5H,m).

[Reference Example 8]

Production of α-methylproline (in the above-mentioned formula (4), $R^1$=Me, $R^2$=$R^3$=H; removal of carbamoylphenylmethyl group, amide hydrolysis)

The crude product (0.50 g) of N-(carbamoylphenylmethyl)-α-methylprolinamide obtained in Example 37, acetic acid (0.070 ml, 1.2 mmol), 10% palladium carbon (PE-type manufactured by N.E. CHEMCAT CORPORATION, 55% water) (29 mg, 0.012 mmol) and methanol (2 ml) were added into a flask, and the mixture was reacted under normal pressure and a hydrogen atmosphere at 60°C for 2 hr. The reaction mixture was filtered through celite, and concentrated to give a crude product of α-methylprolinamide. To the crude product were added water (1 ml) and sulfuric acid (0.26 ml, 4.9 mmol), and the mixture was refluxed for 5 hr to perform amide hydrolysis. The reaction mixture was analyzed by chiral HPLC to find production of a racemate of α-methylproline.

[Example 38]

Production of 1-(carbamoylphenylmethyl)-2-cyano-2-methylpyrrolidine (in the above-mentioned formula (7), $R^1$=Me, $R^2$=carbamoylphenylmethyl, $R^3$=H; step (a) using D-phenylglycinamide hydrochloride, reaction in DMSO solvent)

5-Chloro-2-pentanone (0.25 ml, 2.2 mmol), sodium cyanide (0.29 g, 6 mmol), D-phenylglycinamide hydrochloride (0.37 g, 2 mmol), acetic acid (0.34 ml, 6 mmol), DMSO (1 ml) and water (0.2 ml) were charged in a flask, and the mixture was reacted at room temperature for 6 hr. To the reaction mixture was added water (3.8 ml), and the crystals were collected by filtration, washed with water, and dried under reduced pressure to give 1-(carbamoylphenylmethyl)-2-cyano-2-methylpyrrolidine (0.32 g, 1.3 mmol, yield 67%) as pale-brown crystals. Since derivatization to α-methylproline in the same manner as in Example 37 and Reference Example 8 gave a racemate, it is considered that epimerization of the carbamoylphenylmethyl group proceeded during the reaction.

[Example 39]

Production of 2-phenylpyrroline (in the above-mentioned formula (6), $R^1$=Ph, $R^2$=$R^3$=H; step (a) using 4-chloro-1-phenyl-1-butanone and ammonium acetate, reaction in t-butanol-water solvent)

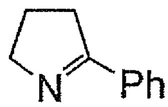

4-Chloro-1-phenyl-1-butanone (0.32 ml, 2 mmol), sodium cyanide (0.11 g, 2.2 mmol), ammonium acetate (0.46 g, 6 mmol), water (1 ml) and t-butanol (2 ml) were charged in a flask, and the mixture was reacted at 70°C for 12 hr. The reaction mixture was extracted with ethyl acetate, and the organic layer was dried over magnesium sulfate. The solvent was evaporated to give a pale-brown oily substance, which was purified by silica gel column chromatography to give 2-phenylpyrroline (87 mg, 0.50 mmol, yield 25%) as a pale-brown oily substance.
$^1$H-NMR (400MHz, $CDCl_3$) $\delta$ 2.00-2.09(2H,m), 2.92-2.99(2H,m), 4.04-4.10(2H,m), 7.38-7.44(3H,m), 7.81-7.87(2H,m).

[Example 40]

Production of 1-benzyl-2-cyano-2-phenylpyrrolidine (in the above-mentioned formula (7), $R^1$=Ph, $R^2$=Bn, $R^3$=H; step (a) using 4-chloro-1-phenyl-1-butanone, benzylamine and acetic acid, reaction in t-butanol-water solvent)

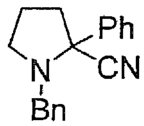

4-Chloro-1-phenyl-1-butanone (0.32 ml, 2 mmol), sodium cyanide (0.11 g, 2.2 mmol), benzylamine (0.66 ml, 6 mmol), acetic acid (0.34 ml, 6 mmol), water (1 ml) and t-butanol (1 ml) were charged in a flask, and the mixture was reacted at 50°C for 9 hr. To the reaction mixture were added 50 w/v% NaOH aqueous solution (0.48 ml) and sodium chloride, and the mixture was extracted with ethyl acetate, and the organic layer was dried over magnesium sulfate. The solvent was evaporated to give a pale-yellow oily substance (1.05 g).

From the results of NMR analysis, this oily substance was a mixture containing 1-benzyl-2-cyano-2-phenylpyrrolidine (45 wt%, 0.47 g, yield 90%), benzylamine (46 wt%) and ethyl acetate (9 wt%).
$^1$H-NMR (400MHz, $CDCl_3$) $\delta$ 1.91-2.28(4H,m), 2.52-2.60(1H,m), 3.16-3.23(1H,m), 3.28(1H,d,J=12.9Hz), 3.74(1H,d,J=13.1Hz), 7.23-7.39(6H,m), 7.41-7.46(2H,m), 7.73-7.77(2H,m).

[Comparative Example 1]

Production of 2-methyl-1-pyrroline (in the above-mentioned formula (6), $R^1$=Me, $R^2$=$R^3$=H; step (a) without addition of sodium cyanide, using aqueous ammonia, reaction in methanol solvent)

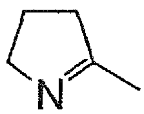

5-Chloro-2-pentanone (0.114 ml, 1.0 mmol), 25% aqueous ammonia (0.34 ml, 5.0 mmol) and methanol (0.34 ml) were charged in a flask, and the mixture was reacted at room temperature for 2 days. The reaction mixture was analyzed based on NMR. As a result, 5-chloro-2-pentanone mostly disappeared but cyclopropyl methyl ketone in an amount almost the same as that of 2-methyl-1-pyrroline was by-produced.

[Comparative Example 2]

Production of 2-methyl-1-pyrroline (in the above-mentioned formula (6), $R^1$=Me, $R^2$=$R^3$=H; step (a) without addition of sodium cyanide, using ammonium acetate, reaction in t-butanol-water solvent)

5-Chloro-2-pentanone (0.114 ml, 1.0 mmol), ammonium acetate (231 mg, 3.0 mmol), water (0.5 ml) and t-butanol (0.5 ml) were charged in a flask, and the mixture was reacted at 50°C for 4 hr. The reaction mixture was analyzed

based on NMR. As a result, 5-chloro-2-pentanone alone was observed, and 2-cyano-2-methylpyrrolidine was not produced.

Industrial Applicability

Optically active $\alpha$-substituted prolines represented by the formula (4) and optically active $\alpha$-substituted prolinamides represented by the formula (5) produced by the method of the present invention are useful as pharmaceutical intermediates.

This application is based on patent application No. 2011-122587 filed in Japan, the entire contents of which are incorporated by reference herein.

## Claims

1. A method of producing an optically active $\alpha$-substituted proline represented by the formula (4)

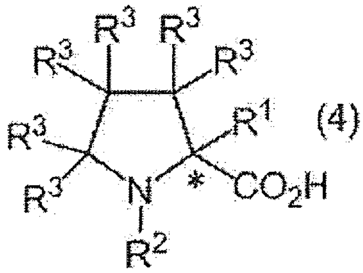

(4)

wherein $R^1$ is an optionally substituted alkyl group, an optionally substituted aryl group, or an optionally substituted heteroaryl group, $R^2$ is a hydrogen atom, an optionally substituted alkyl group, or an amino-protecting group, each $R^3$ is independently a hydrogen atom, an optionally substituted alkyl group, an optionally substituted aryl group, an optionally substituted heteroaryl group, an optionally substituted hydroxyl group, an optionally substituted amino group, an optionally substituted thiol group, or a halogen atom, two or more $R^3$ optionally form one or plural cyclic structures, and * shows an asymmetric carbon, or a salt thereof, and/or an optically active $\alpha$-substituted prolinamide represented by the formula (5)

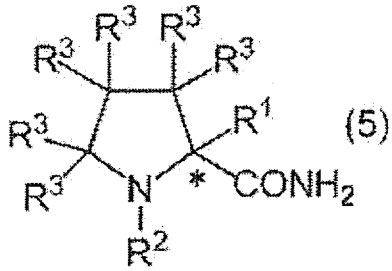

(5)

wherein each symbol is as defined above, or a salt thereof, comprising the following steps (a) to (c);

(a) reacting an acyclic ketone compound represented by the formula (1)

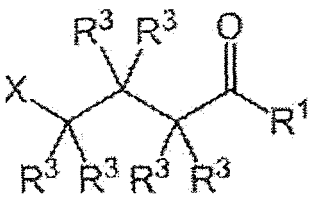

(1)

wherein $R^1$ and $R^3$ are as defined above, and X is a halogen atom or a sulfonyloxy group, with at least one selected from ammonia, an ammonium salt, primary amine and a salt of primary amine, and a cyanating agent and, where necessary, protecting a nitrogen atom on the pyrrolidine ring to give a cyclic nitrogen-containing compound represented by the formula (2)

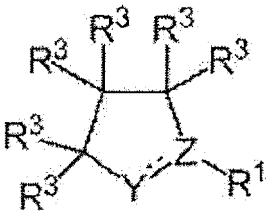

(2)

wherein $R^1$ and $R^3$ are as defined above, Y is a nitrogen atom or a nitrogen atom substituted by $R^2$, Z is a carbon atom or a carbon atom substituted by a cyano group, when Y is a nitrogen atom and Z is a carbon atom, then the bond between Y and Z is a double bond, when Y is a nitrogen atom substituted by $R^2$ and Z is a carbon atom substituted by a cyano group, then the bond between Y and Z is a single bond, and $R^2$ is as defined above, or a salt thereof; and

(b) hydrating the cyclic nitrogen-containing compound represented by the formula (2) or a salt thereof to give an $\alpha$-substituted prolinamide represented by the formula (3)

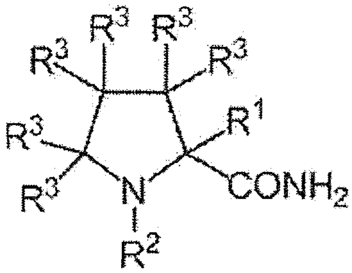

(3)

wherein each symbol is as defined above, or a salt thereof; and (c) resolving the $\alpha$-substituted prolinamide represented by the formula (3) or a salt thereof to give an optically active $\alpha$-substituted proline represented by the formula (4) or a salt thereof, and/or an optically active $\alpha$-substituted prolinamide represented by the formula (5) or a salt thereof.

**2.** A method of producing a 2-substituted prolinamide represented by the formula (3)

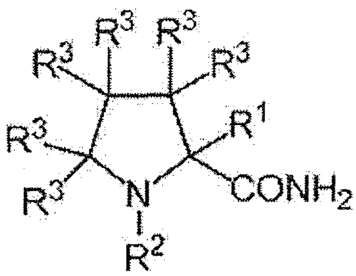

(3)

wherein each symbol is as defined in claim 1, or a salt thereof, comprising the following steps (a) and (b);

(a) reacting an acyclic ketone compound represented by the formula (1)

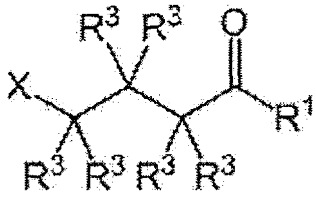

(1)

wherein each symbol is as defined in claim 1, with at least one selected from ammonia, an ammonium salt, primary amine and a salt of primary amino, and a cyanating agent and, where necessary, protecting a nitrogen atom on the pyrrolidine ring to give a cyclic nitrogen-containing compound represented by the formula (2)

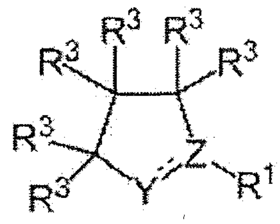

(2)

wherein each symbol is as defined in claim 1, or a salt thereof; and

(b) hydrating the cyclic nitrogen-containing compound represented by the formula (2) or a salt thereof to give an $\alpha$-substituted prolinamide represented by the formula (3) or a salt thereof.

**3.** A method of producing a cyclic nitrogen-containing compound represented by the formula (2)

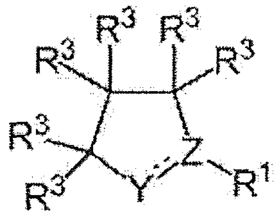

(2)

wherein each symbol is as defined in claim 1, or a salt thereof, comprising the following step (a);

(a) reacting an acyclic ketone compound represented by the formula (1)

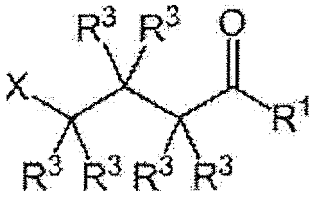

(1)

wherein each symbol is as defined in claim 1, with at least one selected from ammonia, an ammonium salt, primary amine and a salt of primary amine, and a cyanating agent and, where necessary, protecting a nitrogen atom on the pyrrolidine ring to give a cyclic nitrogen-containing compound represented by the formula (2) or a salt thereof.

**4.** A method of producing an optically active $\alpha$-substituted proline represented by the formula (4)

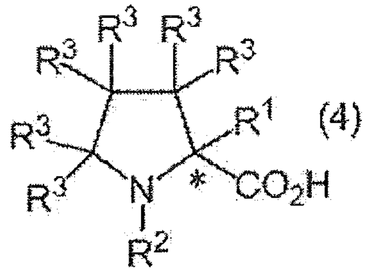

(4)

wherein each symbol is as defined in claim 1, or a salt thereof, and/or an optically active α-substituted prolinamide represented by the formula (5)

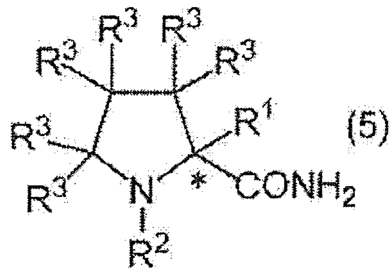

(5)

wherein each symbol is as defined in claim 1, or a salt thereof, comprising the following step (c);

(c) resolving an α-substituted prolinamide represented by the formula (3)

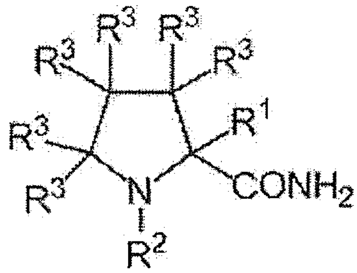

(3)

wherein each symbol is as defined in claim 1, or a salt thereof, to give an optically active α-substituted proline represented by the formula (4) or a salt thereof, and/or an optically active α-substituted prolinamide represented by the formula (5) or a salt thereof;
wherein the resolution is one or more of the following steps (d) to (f):
(d) asymmetric hydrolysis of the amido group by an enzyme having an amidase activity derived from Rhizopus oryzae,
(e) resolution by diastereomeric salt formation,
(f) separation by column chromatography.

**5.** An α-methylprolinamide represented by the formula (8)

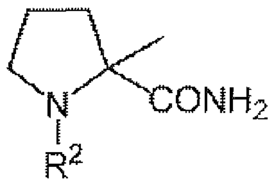 (8)

wherein $R^2$ is 1-phenylethyl group, 1-(1-naphthyl)ethyl group, 1-(2-naphthyl)ethyl group, or carbamoylphenylmethyl group, or a salt thereof.

**INTERNATIONAL SEARCH REPORT**

International application No.
PCT/JP2012/058452

A. CLASSIFICATION OF SUBJECT MATTER
*C07D207/16*(2006.01)i, *C12P41/00*(2006.01)i

According to International Patent Classification (IPC) or to both national classification and IPC

B. FIELDS SEARCHED

Minimum documentation searched (classification system followed by classification symbols)
C07D207/16, C12P41/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | Oleksandr O. Grygorenko, An approach to 2-cyanopyrrolidines bearing a chiral auxiliary, Tetrahedron: Asymmetry, 2007, vol.18, p.290-297 | 5<br>1-3 |
| Y | Jerry Isaacson, Total Synthesis of (±)-Dysibetaine, Organic Letters, 2008, vol.10, p.1461-1463 | 1-3 |
| X<br>Y | Matteo De Poli, Is the Backbone Conformation of C-Methyl Proline Restricted to a Single Region?, Chem. Eur. J., 2009, vol.15, p.8015-8025 | 4<br>1-3 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>26 April, 2012 (26.04.12) | Date of mailing of the international search report<br>15 May, 2012 (15.05.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/058452

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The technical feature common to the invention of claim 1, the invention of claim 4 and the invention of claim 5 is a compound represented by formula (3).

However, the above-said technical feature cannot be considered to be a special technical feature, since the technical feature does not make a contribution over the prior art in the light of the contents disclosed in the document 3.

(Continued to extra sheet)

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2012/058452

Continuation of Box No.III of continuation of first sheet(2)

Furthermore, there is no other same or corresponding special technical feature between the inventions.

The following three invention groups are involved in claims.

(Invention group 1) the inventions of claims 1-3: inventions related to a method for producing a compound represented by formula (2) from a compound represented by formula (1)

(Invention group 2) the invention of claim 4: an invention related to a method for optically resolving a compound represented by formula (3)

(Invention group 3) the invention of claim 5: an invention related to a compound represented by formula (8)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006110816 A
- JP S4931614 A
- WO 2004090152 A
- JP 2011122587 A


### Non-patent literature cited in the description


- *Chem. Eur. J.,* 2009, vol. 15, 8015
- *Org. Synth.,* 1995, vol. 72, 62
- *Synlett,* 1999, 33
- *J. Am. Chem. Soc.,* 2006, vol. 128, 15394
- *J. Am. Chem. Soc.,* 2008, vol. 130, 4153
- *Tetrahedron,* 2010, vol. 66, 4900
- *Tetrahedron Asym.,* 2007, vol. 18, 290
- *Tetrahedron Asym.,* 2006, vol. 17, 252
- *Heterocycles,* 1997, vol. 45, 1447
- *Bull. Soc. Chim. Fr.,* 1986, 83
- *Org. Synth.,* 1951, vol. 31, 74
- *Chem. Pharm. Bull.,* 1989, vol. 37, 958